(19) **Europäisches Patentamt**
**European Patent Office**
**Office européen des brevets**

(11) **EP 3 237 011 B1**

(12) **EUROPEAN PATENT SPECIFICATION**

(45) Date of publication and mention
of the grant of the patent:
**10.11.2021 Bulletin 2021/45**

(21) Application number: **15821075.7**

(22) Date of filing: **22.12.2015**

(51) Int Cl.:
*A61K 38/14* *(2006.01)*     *A61K 31/365* *(2006.01)*
*A61K 31/366* *(2006.01)*    *A61K 31/407* *(2006.01)*
*A61K 31/424* *(2006.01)*    *A61K 31/427* *(2006.01)*
*A61K 31/43* *(2006.01)*      *A61K 45/06* *(2006.01)*
*A61K 9/00* *(2006.01)*      *C12Q 1/18* *(2006.01)*
*G01N 33/50* *(2006.01)*

(86) International application number:
**PCT/EP2015/080933**

(87) International publication number:
**WO 2016/102541 (30.06.2016 Gazette 2016/26)**

(54) **COMPOSITION COMPRISING VANCOMYCIN AND ORLISTAT**

ZUSAMMENSETZUNG ENTHALTEND VANCOMYCIN UND ORLISTAT

COMPOSITION COMPRENNANT VANCOMYCIN ET ORLISTAT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2014 EP 14199908**

(43) Date of publication of application:
**01.11.2017 Bulletin 2017/44**

(73) Proprietor: **Université Libre de Bruxelles
1050 Bruxelles (BE)**

(72) Inventors:
• **FONTAINE, Véronique**
  **3080 Tervuren (BE)**
• **LEFEVRE, Philippe**
  **6900 Marche-en-Famenne (BE)**

(74) Representative: **Savoye, Anne
ICOSA Europe
Rue d'Oultremont 37
1040 Bruxelles (BE)**

(56) References cited:
**WO-A2-01/12168     US-A1- 2010 282 247**

• **GEITER L J ET AL: "United States Public Health Service Tuberculosis Therapy trial 21: Preliminary results of an evaluation of a combination tablet of isoniazid, rifampin and pyrazinamide", TUBERCLE, LONGMAN GROUP UK LTD., HARLOW, GB, vol. 68, 1 June 1987 (1987-06-01), pages 41-46, XP023093868, ISSN: 0041-3879 [retrieved on 1987-06-01]**
• **COLLINS C H ET AL: "IN-VITRO ACTIVITY OF SEVENTEEN ANTIMICROBIAL COMPOUNDS AGAINST SEVEN SPECIES OF MYCOBACTERIA", JOURNAL OF ANTIMICROBIAL CHEMOTHERAPY, OXFORD UNIVERSITY PRESS, GB, vol. 22, no. 6, 1 January 1988 (1988-01-01), pages 857-862, XP009183991, ISSN: 0305-7453**
• **MAJUMDAR ANINDITA ET AL: "Effect of HIV protease inhibitors and Orlistat on mycobacterial ES-31 serine protease, a potential drug target in Mycobacterium tuberculosis.", THE INDIAN JOURNAL OF TUBERCULOSIS JAN 2011, vol. 58, no. 1, January 2011 (2011-01), pages 4-10, XP055186014, ISSN: 0019-5707**
• **GAURI WANKHADE ET AL: "Inhibitory effect of isoniazid and orlistat combination on mycobacterial ES-31 serine protease in vitro and on the growth of M.tb bacilli in axenic culture", INDIAN JOURNAL OF TUBERCULOSIS, vol. 59, no. 3, 1 July 2012 (2012-07-01), pages 156-161, XP055186016, IN ISSN: 0019-5707**

**(Cont. next page)**

- PROVVEDI ROBERTA ET AL: "Global transcriptional response to vancomycin in Mycobacterium tuberculosis", MICROBIOLOGY (READING), vol. 155, no. Part 4, April 2009 (2009-04) , pages 1093-1102, XP002743626, ISSN: 1350-0872
- CHEN FENG-CHI ET AL: "Pros and Cons of the Tuberculosis Drugome Approach - An Empirical Analysis", PLOS ONE, vol. 9, no. 6, June 2014 (2014-06), XP055209267,
- AGERTT VANESSA ALBERTINA ET AL: "Evaluation of antimycobacterial activity of a sulphonamide derivative", TUBERCULOSIS (AMSTERDAM), vol. 93, no. 3, May 2013 (2013-05), pages 318-321, XP055209268, ISSN: 1472-9792
- SCHOONMAKER MAIA K ET AL: "Nonclassical Transpeptidases of Mycobacterium tuberculosis Alter Cell Size, Morphology, the Cytosolic Matrix, Protein Localization, Virulence, and Resistance to beta-Lactams", JOURNAL OF BACTERIOLOGY, vol. 196, no. 7, April 2014 (2014-04), pages 1394-1402, XP002743628,
- NZILA ALEXIS ET AL: "Drug repositioning in the treatment of malaria and TB", FUTURE MEDICINAL CHEMISTRY, LONDON : FUTURE SCIENCE, UK, vol. 3, no. 11, 1 September 2011 (2011-09-01), pages 1413-1426, XP009185837, ISSN: 1756-8927, DOI: 10.4155/FMC.11.95
- CAVALIERI S J ET AL: "Synergistic Activities of Clarithromycin and Antituberculous Drugs against Multidrug-Resistant Mycobacterium tuberculosis", ANTIMICROBIAL AGENTS AND CHEMOTHERAPY, AMERICAN SOCIETY FOR MICROBIOLOGY, vol. 39, no. 7, 1 January 1995 (1995-01-01), pages 1542-1545, XP002311732, ISSN: 0066-4804
- None

**Description**

**FIELD OF INVENTION**

The present invention relates to novel combinations of substances and novel pharmaceutical compositions for treating mycobacterial infections, and medical uses of those combinations and compositions. The present invention further concerns a method for screening new compounds useful for treating mycobacterial infections and/or for treating multidrug resistant (MDR) and Extremely resistant (XDR) mycobacterial strains.

**BACKGROUND OF INVENTION**

Tuberculosis (TB) is a highly contagious disease condition caused by droplet-transmitted infection with *Mycobacterium tuberculosis* (Mtb). Mtb is a facultative intracellular bacterial pathogen that invades and occupies macrophages. According to the World Health Organization (WHO) more than 2 billion people worldwide are infected with *Mycobacterium tuberculosis.* Most of those people have latent tuberculosis, but about 1 in 10 (many of them HIV positive) can expect to develop active TB at some stage in their lifetime. In 2008 1.8 million people died from TB.

Unfortunately, *Mycobacterium tuberculosis* has a high intrinsic resistance to the majority of clinically applied antibiotics, which severely limits treatment options. This intrinsic resistance has been attributed, in part, to its impermeable, hydrophobic cell envelope that acts as a barrier to entry of certain molecules.

The waxy mycolic acid components of the cell envelope are $\alpha$-alkyl $\beta$-hydroxy branched fatty acids existing in the cell wall commonly either as esters of trehalose or esterified to the arabinogalactan core of the bacterial cell wall. Formation of mycolic acids is aided by two Fas (fatty acid synthase) systems and a Pks (type I polyketide synthase). Fas-I, a multifunctional protein, produces short, particularly $C_{16,18}$ fatty acids; Fas-II is a complex of monofunctional proteins that elongates fatty acids generated by Fas-I to yield long-chain fatty acids generally ranging from $C_{48}$ to $C_{64}$ in length. Pks, such as specifically condensase or Pks13 (see Portevin et al. 2004, PNAS 101(1): 314-9) catalyses the condensation of two fatty acids to form mycolic acids. Further, mycolyltransferases are responsible for carrying out esterifications of mycolic acids within the cell wall. Such mycolyltransferase enzymes include *inter alia* the FbpA, FbpB and FbpC proteins of *M. tuberculosis.* Nguyen et al. 2005 (J Bacteriol 187: 6603-11) suggested FbpA as being involved in intrinsic resistance of *M. smegmatis* colonies to certain antibiotics.

Mycolic acids in the cell wall are associated with a number of extractable lipids ("free lipids"), including dimycocerosate esters (DIM). Biosynthesis of DIM involves Fas-I and a number of Pks proteins. Camacho et al. 2001 (J Biol Chem 276: 19845-54) reported that phthiodiolone dimycocerosate mutants of Mtb have an increased permeability for the detergent chenodeoxycholate, supporting the notion that free lipids contribute to the barrier function of the Mtb cell envelope. Sensitivity to certain antibiotics was not affected in those mutants.

Since mycolic acids and free lipids of the mycobacterial cell envelope appear to be key to resisting attack by the host, elements of their biosynthetic pathways have been proposed as targets for discovery of novel drugs against tuberculosis (e.g., FabG1 in WO 03/082911, (3R)-hydroxyacyl-ACP dehydratases in WO 2008/129146, PptT in WO 2007/069089, FbpC in Jackson et al. 1999 (Mol Microbiol 31(5): 1573-1587)). In fact, one of the existing first-line TB drugs (isoniazid) is known to function by targeting the enzyme InhA of the Fas II complex (Marrakchi et al. 2000 (Microbiology 146: 289-96)).

According to current WHO treatment guidelines, first-line drug treatments for TB should be based on isoniazid, rifampicin, pyrazinamide, streptomycin, ethambutol, either singly (for latent infections) or in combination (for active TB). Complete eradication of TB in a patient is difficult because of the need to follow a strict and lengthy drug treatment regimen. Since many patients fail to properly comply with prescribed dosages or complete the course of treatment, drug-resistant Mtb strains are arising ever more frequently. Multidrug-resistant TB (MDR-TB) is defined as TB that is resistant to at least isoniazid and rifampicin (the most effective first-line drugs). Second-line drugs can be employed to target cases of MDR-TB, but these have certain drawbacks, such as greater expense, toxic side-effects, lower efficacy than first-line drugs, or the need for parenteral administration. Second-line drugs include aminoglycosides (e.g. streptomycin, amikacin, kanamycin), polypeptides (e.g. capreomycin), fluoroquinolones, thioamides, cycloserine, and p-aminosalicylic acid. Collins and Uttley (Journal of Antimicrobial Chemotherapy. 1988, 22:857-861) have reported the use of vancomycin against *M. tuberculosis.* Majumdar et al. (Indian J Tuberc. 2011 Jan;58(1):4-10) and Wankhade et al. (Indian J Tuberc. 2012 Jul;59(3):156-61) have reported the inhibitory effect of orlistat on growth of *M. tuberculosis.*

Extensively drug-resistant TB (XDR-TB) occurs when resistance to second-line drugs develops on top of MDR-TB. XDR-TB is defined by the WHO as TB that is resistant to any fluoroquinolone and at least one of three injectable second-line drugs (capreomycin, kanamycin, and amikacin).

Since *M. tuberculosis* is not susceptible to most antibiotics, and the available selection of effective antibiotics is further restricted by the evolution of drug resistance, there is an urgent and unmet need to develop new treatments for TB.

The present inventors have surprisingly discovered that new specific combinations of a first agent preferably selected from glycopeptides and a second agent preferably selected from lipase inhibitors display significant mycobacteriostatic and/or mycobactericidal properties, and thus allows mycobacterial infections to be treated. The present inventors have also surprisingly discovered that such specific combinations display significant mycobacteriostatic and/or mycobactericidal properties towards MDR or XDR mycobacterial strains and thus allow multidrug-resistant or extensively drug-resistant mycobacterial infections to be treated, and provides a new array of combination treatments that can be used as an alternative to established therapies. The present inventors have also surprisingly discovered that glycopeptides alone or such specific combinations can be used for screening new compounds useful for the treatment of mycobacterial infections or for treating MDR or XDR mycobacterial infections.

## SUMMARY

The present invention concerns a composition comprising vancomycin and orlistat.

In a particular embodiment, the composition further comprising at least one additional therapeutic agent. In a more particular embodiment, the at least one additional therapeutic agent is selected in the group consisting of at least one of a glycopeptide antibiotic, a beta-lactam, cycloserine, a RNA synthesis inhibitor, a DNA replication inhibitor, a nucleoside antibiotic, an inhibitor of protein synthesis, an inhibitor of ATP synthesis, an inhibitor of dihydrofolate reductase, a lipase inhibitor, a statin, a protease inhibitor, a heat shock protein inhibitor, a mycolic acid or a fatty acid synthesis inhibitor, an inhibitor of ACP-domain containing proteins, an Ag85C inhibitor, a disulphide bridge inhibitor and a trisubstituted imidazole.

In a particular embodiment, the composition according to the present invention further comprises at least one additional potentiating agent, preferably selected from beta-lactamase inhibitors, wherein said potentiating agent increases the mycobacteriostatic and/or mycobactericidal properties of said composition. In a particular embodiment, the composition of the invention further comprises clavulanate and/or ampicillin as additional potentiating agents.

The present invention further concerns a pharmaceutical composition comprising the composition as defined above and one or more pharmaceutically acceptable excipients.

The present invention further concerns a kit of parts comprising vancomycin and orlistat as described above and further optionally comprising the at least one additional potentiating agent as described above, in a single or in separate vials, together with suitable pharmaceutical excipients, and wherein vancomycin and orlistat, and the optionally at least one additional potentiating agent, are either dissolved or suspended in an acceptable carrier, preferably an aqueous carrier.

The present invention further concerns the composition, the pharmaceutical composition or the kit of parts as described above for use as a medicament.

The present invention further concerns the composition, the pharmaceutical composition or the kit of parts as described above for use in the treatment of a mycobacterial infection, preferably tuberculosis.

## DEFINITIONS

In the present invention, the following terms have the following meanings:

- "**about**", when used to refer to a measurable value such as a parameter, an amount, a temporal duration and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention.

- "**agent**" is meant to encompass any chemical (e.g., inorganic or organic), biochemical or biological substance, molecule or macromolecule (e.g., biological macromolecule), or any combination or mixture thereof, as well as any extract made from biological materials such as bacteria, plants, fungi, or animal cells or tissues. In an embodiment, the term "agent" encompasses nucleic acids, oligonucleotides, ribozymes, peptides, polypeptides, proteins, peptidomimetics, antibodies (including fragments and derivatives thereof), aptamers, chemical substances, preferably organic molecules, more preferably small organic molecules, lipids, carbohydrates, polysaccharides, etc., and any combinations thereof.

- "**Antibiotic agent**" means a mycobacteriostatic agent, i.e. an agent capable to slow down or to stall mycobacteria growth, or a mycobactericidal agent, i.e. capable to kill a mycobacteria.

- By "**modulate**" or "**modulating**", it is meant that the agent for use in the context of the invention is capable of generating a qualitative or quantitative modification, e.g. an increase, an activation, a decrease or an inhibition of a variable that is being modulated. The said modulation may encompass an increase in the value of said variable by at least about 10%, *e.g.,* by at least about 20%, preferably by at least about 30%, *e.g.,* by at least about 40%, more preferably by at least about 50%, *e.g.,* by at least about 75%, even more preferably by at least about 100%, *e.g.,* by at least about 150%, 200%, 250%, 300%, 400% or by at least about 500%, compared to a reference situation without said modulation; or modulation may encompass a decrease or reduction in the value of said variable by at least about 10%, *e.g.,* by at least about 20%, by at least about 30%, *e.g.,* by at least about 40%, by at least about 50%, *e.g.,* by at least about 60%, by at least about 70%, *e.g.,* by at least about 80%, by at least about 90%, *e.g.,* by at least about 95%, such as by at least about 96%, 97%, 98%, 99% or even by 100%, compared to a reference situation without said modulation. In a preferred embodiment, the said modulation of the activity and/or level of a recited entity may be specific or selective, *i.e.,* the activity and/or level of the recited entity may be modulated without substantially altering the activity and/or level of random, unrelated entities.

- "activity" refers to the activity of any of a peptide, polypeptide or protein, and may encompass any biological, biochemical, enzymatic, signalling and/or structural activity thereof. By "modulating the activity", it is thus meant that the level of a given peptide, polypeptide or protein may be modulated by the said first agent either by modulation of its expression and/or by modulation of the expressed peptide, polypeptide or protein.

- "synergy" or "synergistic" or "potentiate", refers to the interaction of two or more agents acting together in a positive way to produce an effect that neither could produce alone. Synergy of combined drugs effects can be quantified by various methodologies, e.g. by using drugs at sub-MICs. According to the published Fractional Inhibitory Concentration (FIC) methodology (Odds, Antimicrobiol. Chem., 2003), the Fractional Inhibitory Concentration of a drug A and of a drug B was evaluated as follows: FIC of drug A = (MIC of drug A in combination)/(MIC of drug A alone); FIC of drug B = (MIC of drug B in combination)/(MIC of drug B alone). For identifying any synergic activity between a drug A and a drug B, the Fractional Inhibitory Concentration Index (FICI) was evaluated as follows: FICI = FIC of Drug A + FIC of drug B. When FICI$\leq$0.5, the drugs A and B are considered to be synergic. When FICI = 1, the combined effect of the drugs A and B is considered to be additive. When FICI$\geq$4, the combined effect of the drugs A and B is considered to be antagonist. Alternatively, according to the 'x/y' methodology (David, J. Antimicrob. Chemother., 2001), synergy was defined as x/y < 0.5 with x being the growth index (GI) value obtained for the vial with the combination of drugs and y being the lowest GI value obtained with any of the single drug used within the combination tested.

- "comprising", "comprises" and "comprised of" are used herein are synonymous with "including", "includes" or "containing", "contains", and are inclusive or openended and do not exclude additional, non-recited members, elements or method steps. These terms also encompass "consisting of' and "consisting essentially of'.

- "**subject**" or "**patient**" are used interchangeably and refer to animals, e.g. human patients.

- "**a subject in need of treatment**" refers to subjects that would benefit from treatment of a given condition. Such subjects may include, without limitation, those that have been diagnosed with said condition, those prone to contract or develop said condition and/or those in whom said condition is to be prevented.

- "treat" or "treatment" are regarded as encompassing both the therapeutic treatment of an already developed disease or condition, as well as prophylactic or preventative measures, wherein the aim is to prevent or lessen the chances of incidence of an undesired affliction, such as to prevent the chances of contraction and progression of the disease or condition. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "**Treatment**" can also mean prolonging survival as compared to expected survival if not receiving treatment.

- "**therapeutically effective dose**" means an amount of the said pharmaceutical composition that brings about a positive therapeutic response with respect to treatment of a patient with mycobacterial infection, upon administration.

## DETAILED DESCRIPTION

This invention relates to a composition having anti-mycobacterial properties and comprising (a) a first antibiotic agent (hereinafter referred to as "first agent") selected in the group consisting of at least one of a glycopeptide antibiotic, a beta-lactam, a RNA synthesis inhibitor, a DNA replication inhibitor, a nucleoside antibiotic, an inhibitor of protein synthesis and an inhibitor of ATP synthesis and (b) a second agent (hereinafter referred to as "second agent") selected in the group consisting of at least one of a lipase inhibitor, a statin, a protease inhibitor, a heat shock protein inhibitor, a mycolic acid or a fatty acid synthesis inhibitor, an inhibitor of ACP-domain containing proteins, an ethambutol synthesis inhibitor and a trisubstituted imidazole.

The following aspects other than a composition, a pharmaceutical composition and a kit of parts comprising vancomycin and orlistat and their use are not according to the invention and are present for illustration purposes only.

*"First antibiotic agent" of the composition*

The composition of the invention comprises a "first antibiotic agent", hereinafter referred to as "first agent".

As disclosed herein, the said first agent comprises an antibiotic agent having an effect against Gram-positive bacteria.

As disclosed herein, the said first agent modulates (directly or indirectly) the activity and/or level of peptidoglycan or of one or more components of peptidoglycan.

As disclosed herein, the said first agent is capable of modulating the activity and/or level of one or more mycobacterial cellular targets (e.g. peptides, polypeptides or proteins, such as enzymes), which participate in the biosynthesis (e.g. biochemical synthesis, transportation and/or assembly), maintenance or catabolism of peptidoglycan or of one or more components of peptidoglycan. In an embodiment, the said first agent is capable of inhibiting peptidoglycan biosynthesis or the biosynthesis of one or more components of peptidoglycan.

Components of peptidoglycan are generally known and may in particularly comprise N-acetylglucosamine (NAM), N-acetylmuramic acid (MurNAc) and N-glycolylmuramic acid (MurNGly) and polymers comprising NAM, MurNAc and MurNGly particularly linked by β-(1,4)-glycosidic bonds; and may further comprise Glycine, L- and/or D-Alanine, D-Glutamate, meso-diaminopimelic acid and L-Lysine, and oligomers thereof typically about 3 to about 5 amino acids long.

Preferred antibiotics as disclosed herein that disrupt peptidoglycan may be those active in the later stages of peptidoglycan layer formation, i.e., those that act after the nascent peptidoglycan monomer has been delivered across the cytoplasmic membrane, such as by the carrier molecule undecaprenol diphosphate. It may be particularly preferred as disclosed herein that the antibiotic directly or indirectly inhibits the transpeptidation step of peptidoglycan formation. It is also particularly preferred as disclosed herein that the antibiotic's site of action is external of the cytoplasmic membrane.

As disclosed herein, said first agent is an agonist of the glycin receptor and more preferably cycloserine.

*Glycopeptide antibiotics*

As disclosed herein, the said first agent for use in the composition of the disclosure belong to the class of glycopeptide antibiotics. Glycopeptide antibiotics are understood to function by sterically inhibiting the formation of backbone glycan chains from the simple subunits as they are extruded through the cytoplasmic membrane, and thereby inhibit the subsequent transpeptidation reaction.

As disclosed herein, glycopeptide antibiotics comprise vancomycin, teicoplanin, telavancin, bleomycin, ramoplanin, decaplanin, oritavancin and dalbavancin. In an embodiment, the said first agent is vancomycin or teicoplanin.

In the present invention, the first agent is vancomycin.

*Beta-lactam antibiotics*

As disclosed herein, the said first agent for use in the composition of the disclosure belong to the class of β-lactam antibiotics, that are understood to inhibit enzymes involved in transpeptidation of peptidoglycan. The said first agent according to the present disclosure may belong to any of the sub-classes of β-lactam antibiotics, such as without limitation penams, cephems, monobactams, carbapenems and penems, carbapenams, clavams, carbacephems, and oxacephems.

As disclosed herein, preferred β-lactam antibiotics comprise penams, preferably penicillin, aminopenicillins (e.g. ampicillin, amoxicillin, bacampicillin, hetacillin, metampicillin, talampicillin, epicillin or pivampicillin), carboxypenicillins (e.g. carbenicillin, ticarcillin, or temocillin), or andureidopenicillins (e.g. azlocillin, mezlocillin, or pieracillin).

As disclosed herein, preferred β-lactam antibiotics comprise cephems, preferably cephalosporins and cephamycins, and more preferably cefalozin, cefacetrile, cefadroxil, cefalexin, cefaloglycin, cefalonium, cefaloridine, cefalotin, cefapirin, cefatrizine, cefazedone, cefazaflur, cefradine, cefroxadine, ceftezole, cefuroxime, cefaclor, cefamandole, cefminox, cefonicid, cefforanide, cefotiam, cefprozil, cefbuperazone, cefuzonam, cephamycin, cefoxitin, cefotetan, cefmetazole, cefotaxime, cefixime, ceftazidime, ceftriaxone, cefcapene, cefdaloxime, cefdinir, cefditoren, cefetamet, cefmenoxime, cefodizime, cefoperazone, cefpimizole, cefpiramide, cefpodoxime, cefsulodin, cefteram, ceftibuten, ceftiolene, ceftizoxime, oxacephem, flomoxef, latamoxef, cefepime, cefozopran, cefpirome, cefquinone or ceftobiprole.

As disclosed herein, preferred β-lactam antibiotics comprise monobactams, preferably aztreonam, tigemonam, carumonam and nocardicin A.

As disclosed herein, preferred β-lactam antibiotics comprise carbapenems and penems, preferably ertapenem, imipenem, meropenem, doripenem, biapenem, panipenem, razupenem, tebipenem, lenapenem, tomopenem and faropenem.

As disclosed herein, preferred β-lactam antibiotics also comprise carbapenams, clavams, carbacephems, and oxacephems.

*RNA synthesis inhibitors*

As disclosed herein, the said first agent may be a RNA synthesis inhibitor, and more particularly an inhibitor of bacterial DNA-dependent RNA synthesis capable of inhibiting bacterial DNA-dependent RNA polymerase. As disclosed herein, the said first agent is the RNA synthesis inhibitor Rifampicin or a derivative thereof, or fidaxomycin.

*DNA replication inhibitors*

As disclosed herein, the said first agent may be a DNA replication inhibitor, and more particularly an agent inhibiting a topoisomerase and/or a gyrase activity, such as a topoisomerase inhibitor or a gyrase inhibitor. As disclosed herein, said first agent inhibits topoisomerase I, topoisomerase II, topoisomerase IV and/or gyrase. As disclosed herein, said first agent is a quinolone, preferably a fluoroquinolone, or an azamethylquinolone. As disclosed herein, said quinolone is selected form the group comprising cinoxacin, nalidixic acid, oxolinic acid, piromidic acid, pipemidic acid, rosoxacin, ciprofloxacin, enoxacin, fleroxacin, lomefloxacin, nadifloxacin, norfloxacin, ofloxacin, pefloxacin, rufloxacin, balofloxacin, grepafloxacin, levofloxacin, pazufloxacin, sparfloxacin, temafloxacin, tosufloxacin, clinafloxacin, gatifloxacin, gemifloxacin, moxifloxacin, sitafloxacin, trovafloxacin, prulifloxacin, delafloxacin, nemonoxacin, danofloxacin, difloxacin, enrofloxacin, ibafloxacin, marbofloxacin, orbifloxacin, or sarafloxacin.

*Nucleoside antibiotics*

As disclosed herein, the said first agent may be a nucleoside antibiotic, such as caprazamycin.

*Inhibitors of protein synthesis*

As disclosed herein, the said first agent may be an inhibitor of protein synthesis, and more specifically an agent targeting an early step involving the binding of N-formylmethionyl-tRNA to the ribosome. As disclosed herein, said inhibitor of protein synthesis is an oxazolidinone, such as linezolid, posizolid, tedizolid, radezolid, sutezolid (PNU-100480), AZD5847. As disclosed herein, said inhibitor of protein synthesis is an aminoglycoside, such as streptomycin, kanamycin, tobramycin, gentamicin, neomycin, capreomycin, tigecycline or amikacin.

*Inhibitors of ATP synthesis*

As disclosed herein, the said first agent may be an inhibitor of ATP synthesis, and is preferably a darylquinoline, such as bedaquiline (TMC207 or R207910).

*Phenothiazine or derivatives thereof*

As disclosed herein, the said first agent may be a phenothiazine or derivatives thereof, such as chlorpromazine, promazine, triflupromazine, levopromazine, methotripremazine, mesoridazine, thioridazine, fluphenazine, perphenazine, prochlorperazine or trifluoperazine.

*Inhibitors of dihydrofolate reductase*

As disclosed herein, the said first agent may be an inhibitor of dihydrofolate reductase, and more preferably para-aminosalicylic acid (also known as 4-aminosalicylic acid or PAS).

**"Second agent" of the composition**

The composition of the invention further comprises a "second agent", which is acting synergically with said first antibiotic agent.

*Lipase inhibitors*

As disclosed herein, said "second agent" is a lipase inhibitor.

As disclosed herein, said lipase inhibitor is orlistat, also known as tetrahydrolipstatin, or THL, IUPAC name (S)-((S)-1-((2S,3S)-3-Hexyl-4-oxooxetan-2-yl)tridecan-2-yl) 2-formamido-4-methylpentanoate; CAS Registry number 96829-58-2.

In the present invention, the second agent is orlistat.

*Statins*

As disclosed herein, said second agent is a HMG-CoA reductase inhibitor, preferably at least one statin selected from the group comprising atorvastatin, cerivastatin, fluvastatin, lovastatin, mevastatin, pitavastatin, pravastatin, rosuvastatin and simvastatin or mixtures thereof.

As disclosed herein, said second agent is simvastatin, IUPAC name (1S,3R,7S,8S,8aR)-8-{2-[(2R,4R)-4-hydroxy-6-oxotetrahydro-2H-pyran-2-yl]ethyl}-3,7-dimethyl-1,2,3,7,8,8a-hexahydronaphthalen-1-yl 2,2-dimethylbutanoate; CAS Registry number 79902-63-9.

*Protease inhibitors*

As disclosed herein, said second agent is a protease inhibitor, and preferably a HIV-protease inhibitor. Preferred protease inhibitors for use in the composition of the present disclosure comprise ritonavir, lopinavir and indinavir, preferably ritonavir.

As disclosed herein, the said second agent is ritonavir, IUPAC name 1,3-thiazol-5-ylmethyl N-[(2S,3S,5S)-3-hydroxy-5-[(2S)-3-methyl-2-{[methyl({[2-(propan-2-yl)-1,3-thiazol-4-yl]methyl})carbamoyl]amino}butanamido]-l,6-diphenylhexan-2-yl]carbamate; CAS Registry number 155213-67-5.

*Heat shock protein inhibitors*

As disclosed herein, said second agent is a heat shock protein inhibitor, and preferably an inhibitor of heat shock protein HSP90. As disclosed herein, heat shock protein inhibitors for use in the present disclosure are selected from geldanamycin, radicicol and derivatives thereof.

*Mycolic acid or fatty acid synthesis inhibitors*

As disclosed herein, said second agent is a mycolic acid or a fatty acid synthesis inhibitor. In an embodiment, said second agent is in particular capable of disrupting a mycobacterial cell wall, i.e. it is capable of disrupting any of the plasma membrane, the peptidoglycan layer, the arabinogalactan layer, the mycolic acid layer and/or the capsule layer of a mycobacteria. As disclosed herein, said second agent disrupts the mycolic acid layer of a mycobacteria, through its action directed to the mycolic acids and/or the intercalated free lipids of said mycolic acid layer. As disclosed herein, said second agent disrupts the layer formed of mycolic acids or salts and esters thereof, and/or the layer formed of dimycocerosate esters (DIM), preferably of dimycocerosate esters of phthiocerol (PDIM) or glycosyl phenol phthiocerols. As disclosed herein, said second agent modulates the activity and/or the levels of one or more components involved in the formation of the layer of mycolic acids or salts and esters thereof, and/or of the layer of dimycocerosate esters (DIM), preferably of dimycocerosate esters of phthiocerol (PDIM) or glycosyl phenol phthiocerols. As disclosed herein, said second agent modulates the activity and/or levels of one or more mycobacterial cellular targets which participate to the biosynthesis, maintenance or catabolism of the free lipids and/or mycolic acids forming the mycolic acid layer. As disclosed herein, said second agent specifically binds to one or more components of mycolic acid layer, and more particularly to the mycolic acids, or salts and esters thereof, and/or to the free lipids such as dimycocerosate esters (DIM), dimycocerosate esters of phthiocerol (PDIM) or glycosyl phenol phthiocerols. As disclosed herein, said second agent specifically binds to one or more mycobacterial targets which participate in the biosynthesis, maintenance or catabolism of the free lipids and/or mycolic acids forming the mycolic acid layer. As disclosed herein, said second agent is inhibits the biosynthesis of mycolic acids or salts and esters thereof, or of dimycocerosate esters (DIM), preferably of dimycocerosate esters of phthiocerol (PDIM) or glycosyl phenol phthiocerol.

As disclosed herein, the said second agent may be a chemical compound, and more specifically a chemical compound that can inhibit the mycolic acid synthesis and thus disrupt the mycolic acid layer. Chemical compounds for use as second agent within the context of the present disclosure comprise, *inter alia,* thiolactomycin, cerulenin, thiocarbamides, hydrazides, Ag85c inhibitor or biomolecules.

*Thiolactomycin*

As disclosed herein, the second agent for use in the composition of the disclosure is Thiolactomycin or its analogues (such as those disclosed in Douglas JD et al. Microbiology 148 (2002): 3101-3109).

*Cerulenin*

As disclosed herein, the second agent is Cerulenin, i.e. a chemical compound having the IUPAC name (2R,3S)-3-[(4E,7E)-nona-4,7-dienoyl]oxirane-2-carboxamide; CAS Registry number 17397-89-6.

*Thiocarbamides*

As disclosed herein, the second agent is a thiocarbamide, preferably selected from the group comprising ethionamide, prothionamide, and thiourea.

*Hydrazides*

As disclosed herein, said second agent is isoniazid, or laniazid, nydrazid, isonicotinylhydrazine or INH, CAS Registry number 54-85-3. Isoniazid is in particular known to be a prodrug that has to be activated by a bacterial catalase-peroxidase enzyme called KatG. in *M. tuberculosis.* KatG couples the isonicotinic acyl with NADH to form isonicotinic acyl-NADH complex. This complex binds tightly to the enoyl-acyl carrier protein reductase known as InhA, thereby blocking the natural enoyl-AcpM substrate and the action of fatty acid synthase. This process is in particular capable of inhibiting the synthesis of mycolic acid, required for the mycobacterial cell wall to be formed.

*Ag85c inhibitors*

As disclosed herein, said second agent is an inhibitor of the antigen 85C (Ag85C), a mycolyltransferase found in *M. tuberculosis.*

*Inhibitors of ACP-domain containing proteins*

As disclosed herein, said second agent is an inhibitor acyl carrier protein (ACP), which represents an important component in both fatty acid and polyketide biosynthesis with the growing chain bound during synthesis as a thiol ester at the distal thiol of a 4'-phosphopantetheine moiety. Inhibitors of ACP-domain containing proteins as disclosed herein comprise aloe-emodin, an anthraquinone present in aloe latex and nimbin, a chemical compound classified as a triterpenoid isolated from *Azadirachta indica.*

*Trisubstituted imidazoles*

As disclosed herein, said second agent is a trisubstituted imidazole acting as inhibitor of the mycobacterial glutamine synthetase. Trisubstituted imidazoles as disclosed herein for instance include 2-*tert*-butyl-4,5-diarylimidazoles.

*Disulfide bridge inhibitors*

As disclosed herein, said second agent is a disulfide bridge inhibitor, comprising in particular acetylcystein or carbocystein.

*Biological molecules*

As disclosed herein, said second agent is a biological molecule selected from the group consisting of a nucleic acid, an oligonucleotide, a ribozyme, a peptide, a polypeptide, a protein, an antibody, a peptidomimetic, an antibody or a fragment or derivative thereof, and an aptamer.
As disclosed herein, said second agent may be an antisense agent (e.g., antisense oligonucleotides) or a RNA interference agent (e.g., siRNA or shRNA) or a ribozyme or constructs or vectors encoding any such agents. For example, increased expression may be achieved by administering recombinant nucleic acids encoding a desired peptide, polypeptide or protein under the control of a suitable promoter. For example, the level of a peptide, polypeptide or protein may be modulated via alteration of its formation (such as, e.g., folding, interactions), stability, degradation, cellular localisation, etc. By "antisense", it is meant a molecule designed to interfere with gene expression and capable of specifically binding to an intended target nucleic acid sequence. Antisense agents are well known to the one of skill in the art and typically encompass an oligonucleotide or oligonucleotide analogue capable of specifically hybridising to the target sequence, and may typically comprise, consist essentially of or consist of a nucleic acid sequence that is complementary or substantially complementary to a sequence within genomic DNA, mRNA or cDNA, preferably mRNA or cDNA corresponding to the target nucleic acid. Antisense agents suitable herein may typically be capable of hybridising to their respective target at high stringency conditions, and may hybridise specifically to the target under physiological conditions.

Within the context of the disclosure, by "ribozyme" it is meant a nucleic acid molecule, preferably an oligonucleotide or oligonucleotide analogue, capable of catalytically cleaving a polynucleotide. Preferably, a "ribozyme" as disclosed herein may be capable of cleaving mRNA of a given target protein, thereby reducing translation thereof.

A skilled reader shall appreciate whether modulation should result in an increased or reduced activity and/or level of a recited entity. By means of example and not limitation, reducing the activity and/or level of mycobacterial cellular targets (e.g., peptides, polypeptides or proteins, such as enzymes) which participate in the biosynthesis or maintenance of the mycobacterial cell wall, more specifically in the biosynthesis or maintenance of the mycolic acid layer and even more specifically in the biosynthesis or maintenance of the free lipid layer or of the mycolic acids layer, or of component(s) thereof may result in disrupting said layer(s).

Within the context of the disclosure, by "specifically binding", it is meant that the said second agent binds to one or more desired targets or ligands of interest substantially to the exclusion of other molecules which are random or unrelated, and optionally substantially to the exclusion of other molecules that are structurally related. Binding of an agent to a target may be evaluated *inter alia* using conventional interaction-querying methods, such as co-immunoprecipitation, immunoassay methods, chromatography methods, gel electrophoresis methods, yeast two hybrid methods, or combinations thereof. It is not required that the said agent binds exclusively to its intended target(s) or ligand(s). For example, the said second agent as disclosed herein may be said to specifically bind to a target if its affinity for such intended target under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

As disclosed herein, the said second agent may bind to its intended target(s) with affinity constant ($K_A$) of such binding $K_A \geq 1 \times 10^6$ M$^{-1}$, more preferably $K_A \geq 1 \times 10^7$ M$^{-1}$, yet more preferably $K_A \geq 1 \times 10^8$ M$^{-1}$, even more preferably $K_A \geq 1 \times 10^9$ M$^{-1}$, and still more preferably $K_A \geq 1 \times 10^{10}$ M$^{-1}$ or $K_A \geq 1 \times 10^{11}$ M$^{-1}$.

As disclosed herein, the said second agent is an "antibody", and may be any of a monoclonal antibody, a polyclonal antibody, a multispecific antibody (e.g. bispecific antibody), and antibody fragments, so long as they exhibit the desired biological activity. As disclosed herein, the said antibody may be formed by any of a chimeric or a humanized antibody, or may correspond to any antigen-binding portion (fragment) of an intact antibody that retain capacity to specifically bind the antigen of the intact antibody. Examples of antigen-binding portions in particular include (i) a Fab fragment; (ii) a F(ab')2 fragment; (iii) a Fd fragment; (iv) a Fv fragment; (v) a single domain antibody ("sdAb", also known as a nanobody®); (vi) an isolated complementarity determining region (CDR), or a single chain Fv (scFv).

As disclosed herein, the said second agent may be an aptamer. By "aptamer", it is meant a single-stranded or double-stranded oligo-DNA, oligo-RNA or oligo-DNA/RNA or any analogue thereof that can specifically bind to a target molecule. Aptamers generally advantageously display fairly high specificity and affinity (e.g., $K_A$ in the order $1 \times 10^9$ M$^{-1}$) for their targets. The term "photoaptamer" refers to an aptamer that contains one or more photoreactive functional groups that can covalently bind to or crosslink with a target molecule.

As disclosed herein, the said second agent may be a peptidomimetic. By "peptidomimetic", it is meant a non-peptide agent that is a topological analogue of a corresponding peptide.

As disclosed herein, the said second agent is a nucleic acid or a polynucleotide. By "nucleic acid" or "polynucleotide", it is meant a polymeric molecule having a backbone that supports bases capable of hydrogen bonding to typical polynucleotides, where the polymer backbone presents the bases in a manner to permit such hydrogen bonding in a sequence specific fashion between the polymeric molecule and a typical polynucleotide (e.g., single-stranded DNA). Such bases are typically inosine, adenosine, guanosine, cytosine, uracil and thymidine. Polymeric molecules include double and single stranded RNA and DNA, and backbone modifications thereof, for example, methylphosphonate linkages. Polynucleotides may be linear or circular, and include plasmids, viruses and other vectors. Viral vectors include those based on retroviruses, adenoviruses, adeno-associated viruses (AAV), herpes viruses, astroviruses, coronaviruses, orthomyxoviruses, papovaviruses, paramyxoviruses, parvoviruses, picornaviruses, poxviruses, and togaviruses.

Preferred mycobacterial cellular targets participating in the biosynthesis, maintenance or catabolism of the mycolic acid layer or components thereof as disclosed herein include without limitation members of Fas (fatty acid synthase) systems, and including all those reviewed by Takayama et al. Clin. Microbiol. Reviews (2005), p. 81-101. Examples of the genes encoding these targets include: fas (Rv2524c), fabD (Rv2243), acpM (Rv2244), fabH (0533c), fabZ, fabA, kasA/kasB (Rv2245/Rv2246), fabG1, inhA (Rv1784), mmaA1-4 (Rv0642c-Rv0645c), pcaA (Rv0470c), accD4/accd5 (Rv3299c-Rv0380), fadD32 (Rv3801c), pks13 (Rv3800c), fbpA (Rv3804), fbpB (Rv1886) and fbpC (Rv0129c). Preferably herein, the activity and/or level of said targets is to be reduced.

Preferred mycobacterial cellular targets participating in the biosynthesis, maintenance or catabolism of the free lipid layer or components thereof as disclosed herein include without limitation members of the polyketide synthase systems, including all those reviewed by Onwueme et al. Progress in Lipid Research 44 (2005), 259-302. Examples of the genes encoding these targets include: any of the pks family of genes, e.g. ppsA, ppsB, ppsC, ppsD, ppsE, pks6, pks13, mas, pks15/1, pks2, pks3/4, pks7, pks8/17, pks9, pks12, pks10, pks11, pks16. Examples of genes encoding other potential targets include: papA5, fadD26, fadD28, mmpL7, drrABC, 1ppX, and hsp60-1. Preferably herein, the activity and/or level of said targets are to be reduced.

### Additional therapeutic agent

In one embodiment, the composition of the invention further comprising at least one additional therapeutic agent.

In one embodiment, said additional therapeutic agent is selected in the group comprising or consisting of at least one of a glycopeptide antibiotic, a beta-lactam, cycloserine, a RNA synthesis inhibitor, a DNA replication inhibitor, a nucleoside antibiotic, an inhibitor of protein synthesis, an inhibitor of ATP synthesis, an inhibitor of dihydrofolate reductase, a lipase inhibitor, a statin, a protease inhibitor, a heat shock protein inhibitor, a mycolic acid or a fatty acid synthesis inhibitor, an inhibitor of ACP-domain containing proteins, an Ag85C inhibitor, a disulphide bridge inhibitor and a trisubstituted imidazole, as described hereinabove, or mixtures thereof.

### Additional potentiators

In an embodiment, the composition of the invention comprises vancomycin and orlistat, and further comprises at least one additional agent possibly selected in the categories described above for any of the first or second agent, or acting as a potentiator of vancomycin or orlistat. In an embodiment, said additional agent corresponds to a β-lactamase inhibitor, and is capable of potentiating the effects of a first agent selected in the class of β-lactam antibiotics. In a preferred embodiment, beta-lactamase inhibitors for use as potentiators in the present invention comprise penicillins, ceftaroline, imipenem, temocillin, aztreonam, tazobactam or Novexel (also known as NXL104). The composition of the invention may further comprise potentiators selected from telithromycin, piperacillin or cilastatin.

### Specific Combinations of first and second agents

In the present invention, the composition comprises vancomycin and orlistat.

In the present invention, the composition comprises vancomycin and orlistat, wherein vancomycin and orlistat act synergically.

In an embodiment, the composition of the invention is a pharmaceutical composition. In an embodiment, said pharmaceutical composition comprises said first agent (a) and said second agent (b) together in a single dosage form with one or more pharmaceutically acceptable excipients. Alternatively said first agent (a) and said second agent (b) may be formulated in separate pharmaceutical compositions with one or more pharmaceutical excipients. In an embodiment of the invention, the said first and second agents are intended for being administered separately, sequentially or simultaneously to a patient.

Depending on the selected mode of administration, the pharmaceutical composition of the invention may be conditioned under any suitable dosage form known to the skilled person in the art, such as tablets, capsules, lozenges, powders, suppositories, solutions, suspensions, sprays, aerosols, lotions, creams, and dermal patches.

When the pharmaceutical composition of the invention is intended to be conditioned in liquid dosage forms, a variety of aqueous carriers may be used, e.g., water, buffered water, 0.8% saline, phosphate-containing solutions, 0.3% glycine, hyaluronic acid and the like. These compositions may be sterilized by conventional sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, preservatives, tonicity adjusting agents, wetting agents and the like, for example, sodium acetate, sodium lactate, sodium chloride, potassium chloride, calcium chloride, sorbitan monolaurate, triethanolamine oleate, etc. Preferably, the pH value of the composition is in the physiological pH range, such as particularly the pH of the composition is between about 5 and about 9.5, more preferably between about 6 and about 8.5, even more preferably between about 7 and about 7.5. The preparation of such pharmaceutical compositions is within the ordinary skill of a person skilled in the art.

In another particular embodiment of the invention, when the pharmaceutical composition is intended for being used under solid form, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like.

The pharmaceutical composition of the invention may be administered by any route known to the skilled person in the art. The said composition may thus be administered by parenteral, topical, oral or local administration. They may be administered, for example, by the intramuscular, intravenous, intraperitoneal, subcutaneous, transdermal, intradermal, transmucosal, vaginal, rectal, pulmonary, buccal and nasal routes. In an embodiment, the pharmaceutical compositions of the invention are administered parenterally, e.g., intravenously, subcutaneously, intradermally, or intramuscularly.

In an embodiment, the administration is pulmonary administration. In a specific embodiment, the pharmaceutical composition of the invention is administered by inhalation. In an embodiment, the pharmaceutical composition of the invention is administrated via a nebulizer. In a specific embodiment, the pharmaceutical composition of the invention is administrated via an inhalator.

In an embodiment, the composition is administrated as an aerosol. For aerosol administration, the pharmaceutical composition (or the separate components thereof) are preferably supplied in finely divided form along with a surfactant and propellant, in the form of a solution or suspension, optionally with other conventional pharmaceutical excipients. For aerosol administration, appropriate therapeutically effective doses of the said first and second agents in a pharmaceutical composition of the invention can be determined by a qualified physician.

The present invention thus further concerns a kit of parts comprising the said first agent and the said second agent in a single or in separate vials, together with suitable pharmaceutical excipients, and wherein the said first and second agents are either dissolved or suspended in an acceptable carrier, preferably an aqueous carrier. In an embodiment, the invention thus concerns a unit dosage of the pharmaceutical composition of the invention in a suitable amount of an aqueous solution, such as 0.1-3 ml, preferably 0.2-2 mL. The parenteral composition of the invention can be enclosed in ampoules, disposable syringes, or multiple dose vials made of glass or plastic.

The pharmaceutical composition of the invention may be administered within a therapeutically effective dose to a patient in need thereof.

Appropriate therapeutically effective doses of the said first and second agents in a pharmaceutical composition of the invention depend on the nature of the said agents, and can be determined by a qualified physician. Preferred dosages per kg body weight of the patient to be treated may be from about 0.1 to about 100 mg, more preferably from about 0.5 to about 50 mg, and most preferably from about 1 to about 30 mg.

In a particular embodiment, the concentration of orlistat in the composition of the invention is such that the plasmatic concentration of orlistat in the treated patient ranges from about 0.1 ng/mL to about 1 $\mu$g/mL, and preferably from about 1 ng/mL to about 50 ng/mL.

In a particular embodiment, the amount of vancomycin administrated to the patient is from about 500 to about 1000 mg/day, preferably from about 100 to about 500 mg/day, more preferably from about 250 to about 500 mg/day. In a particular embodiment, the amount of vancomycin administrated to the patient is from about 1 to about 50 mg/kg, preferably from about 5 to about 20 mg/kg, more preferably from about 10 to about 20 mg/kg.

In a particular embodiment, the concentration of vancomycin in the composition of the invention is such that the plasmatic concentration of vancomycin in the treated patient ranges from about 1 ng/mL to about 1 mg/mL, and preferably from about 1 $\mu$g/mL to about 100 $\mu$g/mL.

In an embodiment where the composition is administrated as an aerosol, the vancomycin concentration in the liquid medium is from about 25 to about 400 mg/mL, preferably from about 30 to about 120 mg/mL, more preferably from about 50 to about 60 mg/mL. In an embodiment where the composition is administrated as an aerosol, the amount of vancomycin delivered to the lungs or/and pulmonary system is from about 10 to about 1000 mg, preferably from about 40 to about 600 mg.

As disclosed herein, when meropenem is used a first agent, the concentration of meropenem in the composition of the invention is such that the plasmatic concentration of meropenem in the treated patient ranges from 1 ng/mL to 1 mg/mL, and preferably from 1 $\mu$g/mL to 150 $\mu$g /mL.

In a particular embodiment, when clavulanate is used as additional agent, the concentration of clavulanate in the composition of the invention is such that the plasmatic concentration of clavulanate in the treated patient ranges from 1 ng/mL to 1 mg/mL, and preferably from 100 ng/mL to 100 $\mu$g/mL, more preferably from 200 ng/mL to 10 $\mu$g/mL.

In a particular embodiment, when amoxicillin is used as additional agent, the concentration of amoxicillin in the composition of the invention is such that the plasmatic concentration of amoxicillin in the treated patient ranges from 1 ng/mL to 1 mg/mL, and preferably from 100 ng/mL to 100 $\mu$g/mL, more preferably from 1 $\mu$g/mL to 10 $\mu$g/mL.

As disclosed herein, when the said second agent comprised in the pharmaceutical composition is an antibody, the appropriate therapeutically effective doses of said therapeutic antibody can be determined by a qualified physician with due regard to the nature (including half-life) of the antibody, the disease condition and severity, and the age, size and condition of the patient. As disclosed herein, a therapeutically effective dose of the antibody is preferably in the range from about 0.01 mg/kg to about 40 mg/kg, more preferably from about 0.1 mg/kg to about 30 mg/kg, optionally from about 3 mg/kg to about 20 mg/kg.

As disclosed herein, when the said second agent comprised in the pharmaceutical composition is a polypeptide antigen, appropriate therapeutically effective doses of said polypeptide antigen in a pharmaceutical composition according to the invention can be determined by a qualified physician, but generally range for the initial immunization from about 1.0 µg to about 5,000 µg of peptide for a 70 kg patient. The actual dose administered to a subject is often determined according to an appropriate amount per kg of the subject's body weight. For example, an effective amount may be about 0.1 to 5 µg /kg body weight.

As disclosed herein, when the said second agent comprised in the pharmaceutical composition is a polynucleotide vector, appropriate therapeutically effective doses of said polynucleotide vector in a said pharmaceutical composition depends largely on the mode of delivery, but can be determined by a qualified physician, Generally the dose will be from about 0.01 mg to 50 mg per kg body weight of the individual to which it is administered.

In an embodiment, the anti-mycobacterial combinations, compositions, kits and treatments as taught herein may optionally be combined with substances, compositions and treatments aimed at enhancing or restoring compromised immune system. Subjects having compromised immune system, including inter alia asymptomatic HIV-infected (seropositive) subjects and symptomatic AIDS patients, often contract and/or develop mycobacterial infections, such as TB. Accordingly, the present anti-mycobacterial pharmaceutical compositions, kits and treatments may be suitably combined with an anti-retroviral therapy, preferably an anti-HIV therapy, more preferably with a tri-therapy for HIV positive patients. Preferably, such combination does not substantially reduce the respective efficacies of the constituent treatments.

Within the context of the present invention, the terms "subject" or "patient" are used interchangeably and refer to animals, preferably warm-blooded animals, more preferably vertebrates, even more preferably mammals, still more preferably primates, and specifically includes human patients and non-human mammals and primates. Preferred patients are human subjects.

Mycobacterial infections as intended herein encompass infections of subjects with any *Mycobacterium* species, particularly with a pathogenic *Mycobacterium* species, more particularly with a *Mycobacterium* species that is capable of causing tuberculosis and lung disease in subjects, including immune-compromised subjects, such as for example HIV-positive patients (e.g., Mbt complex). Aside from *M. tuberculosis, Mycobacterium* species as intended herein may include without limitation *M. africanum, M. bovis, M. bovis* BCG, *M. avium, M. canetti, M. caprae, M. celatum, M. genavense, M. haemophilum, M. intracellulare, M. kansasii, M. leprae, M. marinum, M. microti, M. simiae, M. ulcerans, M. vaccae, M. fortuitum, M. pinnipedii* and *M. scrofulaceum.* The present invention is particularly directed to infection with *M. tuberculosis.*

Infections treatable according to the present invention include active infections and latent infections. "Active infections" refers to infections with manifested disease symptoms and/or tubercular lesions. "Latent infections" refers to infections without manifested disease symptoms and/or tubercular lesions. The tuberculosis disease condition may be "primary tuberculosis", which refers to clinical illness directly following infection, or it may be "secondary tuberculosis", which refers to the reactivation of a latent infection. The treatment may also be used to prevent or delay TB reactivation. These conditions are described in details in Harrison's Principles of Internal Medicine, Chapter 150, pp. 953-966 (16th ed., Braunwald et al. eds., 2005).

The present invention further discloses a method for assessing the susceptibility of multi-drug resistant (MDR) and extremely drug-resistant (XDR) mycobacterial strains to glycopeptides, beta-lactams or a combination thereof, comprising the steps of:

(a) contacting a MDR or XDR mycobacterial strain with a glycopeptide, a beta-lactam or a combination thereof;

(b) comparing the growth rates of the said MDR or XDR mycobacterial strain in presence and in absence of said glycopeptide, beta-lactam or combination thereof.

As disclosed herein, the method is for assessing the susceptibility of multi-drug resistant (MDR) and extremely drug-resistant (XDR) mycobacterial strains to glycopeptides, and comprises the steps of:

(a) contacting a MDR or XDR mycobacterial strain with a glycopeptide;

(b) comparing the growth rates of the said MDR or XDR mycobacterial strain in presence and in absence of said glycopeptide.

As disclosed herein, the method is for assessing the susceptibility of multi-drug resistant (MDR) and extremely drug-resistant (XDR) mycobacterial strains to vancomycin, and comprises the steps of:

(a) contacting a MDR or XDR mycobacterial strain with vancomycin;

(b) comparing the growth rates of the said MDR or XDR mycobacterial strain in presence and in absence of said vancomycin.

As disclosed herein, the glycopeptide and/or the beta-lactam for use in the method may be any of the glycopeptide or beta-lactam first agents disclosed *supra.* As disclosed herein, the said glycopeptide and/or beta-lactam is unable to provide mycobacteriostatic and/or mycobactericidal effects when the treated mycobacteria possess a layer formed of mycolic acids or salts and esters thereof, and/or of a layer formed of dimycocerosate esters (DIM), preferably of dimycocerosate esters of phthiocerol (PDIM) or glycosyl phenol phthiocerols. As disclosed herein, the said glycopeptide and/or beta-lactam for use in the method is capable of significantly delaying or hindering the growth of a MDR or XDR mycobacterial strain that does not comprise PDIM, but is on the contrary unable or hardly capable of delaying or hindering the growth of MDR or XDR mycobacterial strains comprising a lipidic cell wall, and more preferably comprising PDIM.

As disclosed herein, glycopeptides for use in the method for assessing the susceptibility of MDR or XDR mycobacterial strains preferably comprise vancomycin or teicoplanin. As disclosed herein, the said glycopeptide is used in a concentration of from about 0.5 to about 500 μg/mL, preferably of from about 1 to about 100 μg/mL, more preferably of from about 5 to about 50 μg/mL.

The assessment method now renders it possible to identify MDR or XDR mycobacteria that are highly susceptible to glycopeptides and/or to beta-lactams. The said method thus avoids patients suffering from mycobacterial infections involving MDR or XDR strains to be treated with inefficient combinations of drugs, and further favours the prevention against the development of MDR or XDR in avoiding unnecessary antibiotic treatments to be performed. As disclosed herein, when a MDR or a XDR mycobacterial strain is found to be sensitive to glycopeptides and/or to beta-lactams, the patient infected by this strain may thus be advantageously treated by said glycopeptide, beta-lactam or combination thereof, in substitution or in combination with usual first or second line treatments.

Within the context of the present disclosure, a MDR or XDR mycobacterial strain is considered as being sensitive to glycopeptides, beta-lactams or combinations thereof when the growth of said mycobacterial strains exposed to said glycopeptides, beta-lactams or combinations thereof is reduced when compared to the growth of the untreated mycobacterial strains at a 1/100 dilution.

The present invention further discloses a method for screening synergistic mycobacteriostatic or mycobactericidal combinations of agents, comprising the steps of:

(a) contacting a multidrug resistant (MDR) or an extremely drug-resistant (XDR) mycobacterial strain with a candidate agent in presence of a glycopeptide, a beta-lactam or a combination thereof, and;

(b) comparing the growth rates of the said MDR or XDR mycobacterial strain exposed to the said glycopeptide, beta-lactam or combination thereof in presence and in absence of said candidate agent.

As disclosed herein, the method is for screening synergistic mycobacteriostatic or mycobactericidal combinations of agents, and comprises the steps of:

(a) contacting a multidrug resistant (MDR) or an extremely drug-resistant (XDR) mycobacterial strain with a candidate agent in presence of a glycopeptide, and;

(b) comparing the growth rates of the said MDR or XDR mycobacterial strain exposed to the said glycopeptide in presence and in absence of said candidate agent.

As disclosed herein, the method is for screening synergistic mycobacteriostatic or mycobactericidal combinations of agents, and comprises the steps of:

(a) contacting a multidrug resistant (MDR) or an extremely drug-resistant (XDR) mycobacterial strain with a candidate agent in presence of vancomycin, and;

(b) comparing the growth rates of the said MDR or XDR mycobacterial strain exposed to said vancomycin in presence and in absence of said candidate agent.

As disclosed herein, the glycopeptide, beta-lactam or combination thereof for use in the screening method may be any of the first agents disclosed *supra.* As disclosed herein, the said glycopeptide and/or beta-lactam is unable to provide mycobacteriostatic and/or mycobactericidal effects when the treated mycobacteria possess a layer formed of mycolic acids or salts and esters thereof, and/or of a layer formed of dimycocerosate esters (DIM), preferably of dimycocerosate esters of phthiocerol (PDIM) or glycosyl phenol phthiocerols. As disclosed herein, the said glycopeptide and/or beta-lactam for use in the method is capable of significantly delaying or hindering the growth of a MDR or XDR mycobacterial strain that does not comprise PDIM, but is on the contrary unable or hardly capable of delaying or hindering the growth of MDR or XDR mycobacterial strains comprising a lipidic cell wall, and more preferably comprising PDIM.

As disclosed herein, glycopeptides for use in the method for assessing the susceptibility of MDR or XDR mycobacterial strains preferably comprise vancomycin or teicoplanin. As disclosed herein, the said glycopeptide is used in a concentration of from about 0.5 to about 500 $\mu$g/mL, preferably of from about 1 to about 100 $\mu$g/mL, more preferably of from about 5 to about 50 $\mu$g/mL.

The screening method now renders it possible to identify new compounds that are highly susceptible to be used in a pharmaceutical composition according to the present invention, i.e. in combination with a first agent as described above, for treating MDR and/or XDR mycobacterial infections. The screening method thus now renders it possible to identify new antimycobacterial 1st and 2nd line drugs combinations. The said method thus provides a cost-effective but extremely powerful tool for screening and identifying new compounds capable of treating MDR and XDR mycobacterial strains.

When the MDR or XDR mycobacterial strains assayed with the screening method are placed into contact with a new compound capable of acting synergistically with the selected glycopeptide, beta-lactam or combination thereof, the growth of said mycobacterial strains exposed to the candidate agent in the presence of the glycopeptide, beta-lactam or combination thereof is reduced when compared to the growth of the strain exposed to the antibiotic in absence of the candidate agent at a 1/100 dilution.

On the contrary, when the MDR or XDR mycobacterial strains assayed with the screening method are placed into contact with a candidate agent which is not capable of acting synergistically with the selected glycopeptide, beta-lactam or combination thereof, the growth of said mycobacterial strains exposed to the candidate agent in the presence of the glycopeptide, beta-lactam or combination thereof is equal or greater than the growth of the strain exposed to the glycopeptide, beta-lactam or combination thereof in absence of the candidate compound at a 1/100 dilution.

## BRIEF DESCRIPTION OF THE DRAWINGS

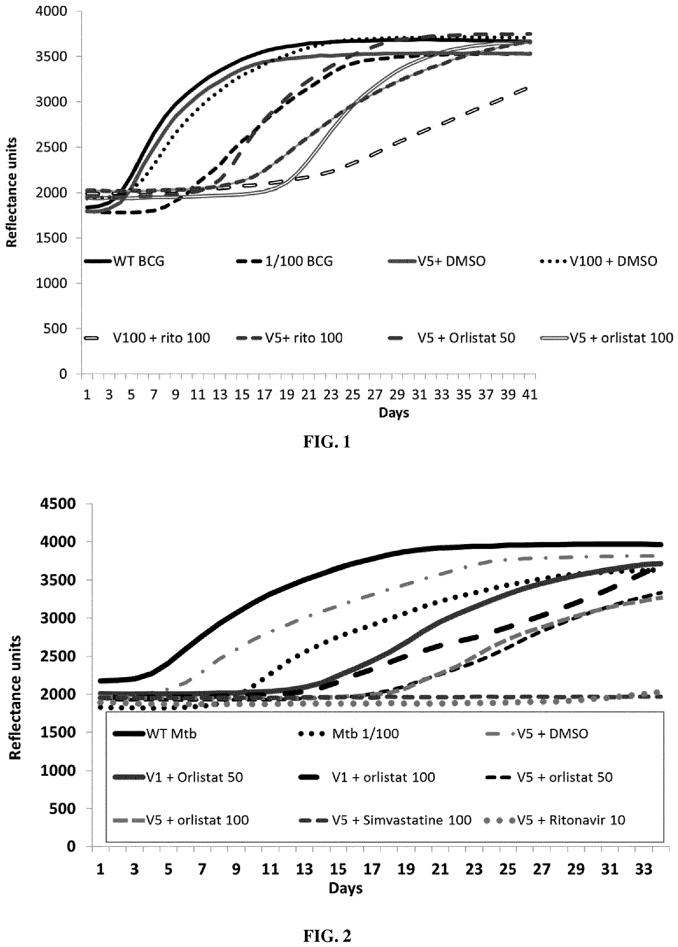

**Figure 1** is a graph showing the impact of several combinations of first and second agents according to the invention on the growth of *Mycobacterium bovis* BCG. The results of Figure 1 were obtained with the BacT/Alert MP system. WT BCG: untreated and undiluted *M. bovis* BCG strain. 1/100 BCG: untreated *M. bovis* strain diluted 1/100. V5+DMSO: *M. bovis* BCG strain grown in presence of vancomycin 5$\mu$g/mL and DMSO. V100+DMSO: *M. bovis* BCG strain grown in presence of vancomycin 100 $\mu$g/mL and DMSO. V100+rito100: *M. bovis* BCG strain grown in presence of vancomycin 100 $\mu$g/mL and ritonavir 100 $\mu$g/mL. V5+rito100: *M. bovis* BCG strain grown in presence of vancomycin 5 $\mu$g/mL and ritonavir 100 $\mu$g/mL. V5+orlistat50: *M. bovis* BCG strain grown in presence of vancomycin 5 $\mu$g/mL and orlistat 50 $\mu$g/mL. V5+orlistat100: *M. bovis* BCG strain grown in presence of vancomycin 5 $\mu$g/mL and orlistat 100 $\mu$g/mL.

**Figure 2** is a graph showing the impact of several combinations of first and second agents according to the invention on the growth of *Mycobacterium tuberculosis* H37Rv. The results of Figure 2 were obtained with the BacT/Alert MP system. WT Mtb: untreated and undiluted *Mycobacterium tuberculosis* H37Rv strain. Mtb 1/100: untreated *M. tuberculosis* strain diluted 1/100. V5+DMSO: *M. tuberculosis* strain grown in presence of vancomycin 5 $\mu$g/mL and DMSO. V1+orlistat 50: *M. tuberculosis* strain grown in presence of vancomycin 1 $\mu$g/mL and Orlistat 50 $\mu$g/mL. V1+orlistat 100: *M. tuberculosis* strain grown in presence of vancomycin 1$\mu$g/mL and Orlistat 100 $\mu$g/mL. V5+orlistat 50: *M. tuberculosis* strain grown in presence of vancomycin 5 $\mu$g/mL and Orlistat 50 $\mu$g/mL. V5+orlistat 100: *M. tuberculosis* strain grown in presence of vancomycin 5 $\mu$g/mL and Orlistat 100 $\mu$g/mL. V1+orlistat 50: *M. tuberculosis* strain grown in presence of vancomycin 1 $\mu$g/mL and Orlistat 50 $\mu$g/mL. V5+simvastatin 100: *M. tuberculosis* strain grown in presence of vancomycin 5 $\mu$g/mL and simvastatin 100 $\mu$g/mL. V5+ritonavir 10: *M. tuberculosis* strain grown in presence of vancomycin 5 $\mu$g/mL and ritonavir 10 $\mu$g/mL.

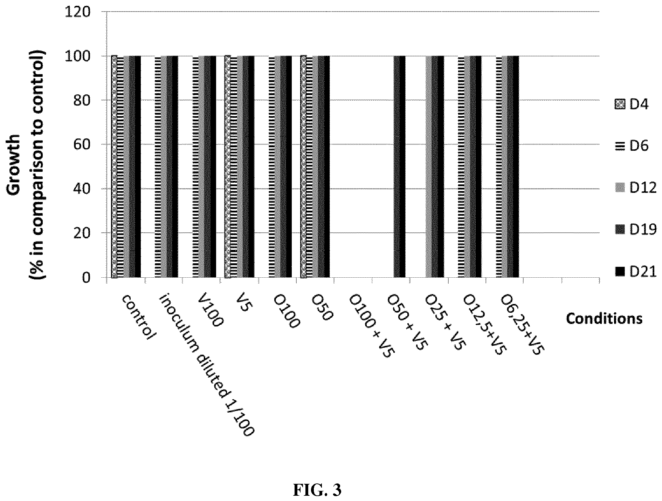

**Figure 3** is a histogram showing the impact of several combinations of first and second agents according to the invention on the growth of *Mycobacterium tuberculosis* H37Rv. The results of Figure 3 were obtained with the standard broth dilution susceptibility test method. Control: untreated and undiluted *M. tuberculosis* strain. Inoculum diluted 1/100: untreated *M. tuberculosis* strain diluted 1/100. V100: *M. tuberculosis* strain grown in presence of vancomycin 100 $\mu$g/mL alone. V5: *M. tuberculosis* strain grown in presence of vancomycin 5$\mu$g/mL alone. O100: *M. tuberculosis* strain grown in presence of orlistat 100 $\mu$g/mL alone. O5: *M. tuberculosis* strain grown in presence of orlistat 5 $\mu$g/mL alone. O100+V5: *M. tuberculosis* strain grown in presence of vancomycin 5 $\mu$g/mL and Orlistat 100 $\mu$g/mL. O50+V5: *M. tuberculosis* strain grown in presence of vancomycin 5 $\mu$g/mL and Orlistat 50 $\mu$g/mL. O25+V5: *M. tuberculosis* strain grown in presence of vancomycin 5 $\mu$g/mL and Orlistat 25 $\mu$g/mL. O12.5+V5: *M. tuberculosis* strain grown in presence of vancomycin 5 $\mu$g/mL and Orlistat 12.5 $\mu$g/mL. O06.25+V5: *M. tuberculosis* strain grown in presence of vancomycin 5 $\mu$g/mL and Orlistat 6.25 $\mu$g/mL. The cell growth is expressed as a percentage in comparison to control. Measurements are performed at days 4, 6, 12, 19 and 21.

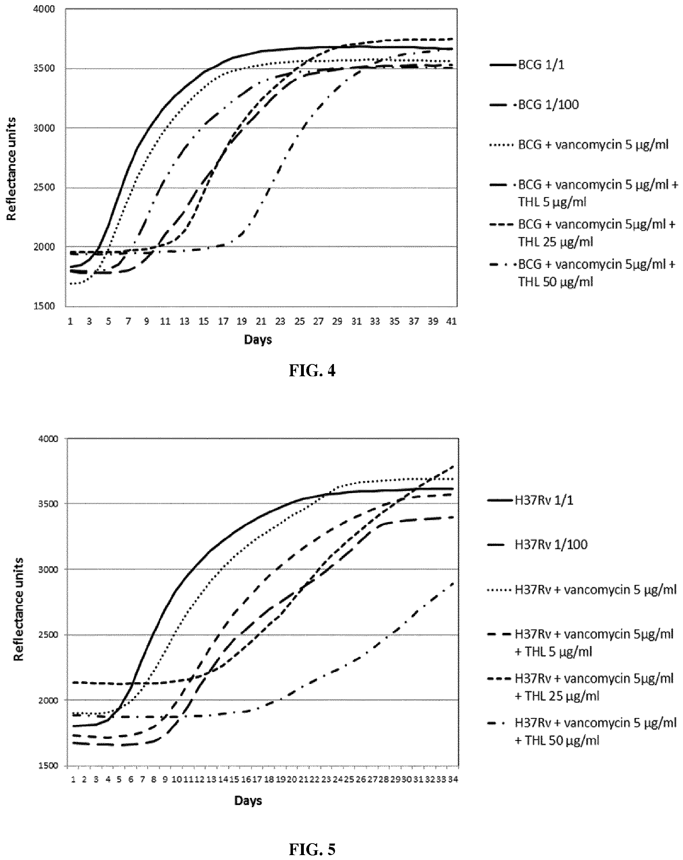

**Figure** 4 is a graph showing the impact of several combinations of vancomycin and orlistat on the growth of *Mycobacterium bovis* BCG. The results of Figure 4 were obtained with the BacT/Alert MP system. BCG 1/1: untreated and undiluted *M. bovis* BCG strain. BCG 1/100: untreated *M. bovis* strain diluted 1/100. BCG + vancomycin 5 μg/ml: *M. bovis* BCG strain grown in presence of vancomycin 5 μg/mL. BCG + vancomycin 5 μg/ml + THL 5 μg/ml: *M. bovis* BCG strain grown in presence of vancomycin 5 μg/mL and orlistat 5 μg/mL. BCG + vancomycin 5 μg/ml + THL 25 μg/ml: *M. bovis* BCG strain grown in presence of vancomycin 5 μg/mL and orlistat 25 μg/mL. BCG + vancomycin 5 μg/ml + THL 50 μg/ml: *M. bovis* BCG strain grown in presence of vancomycin 5 μg/mL and orlistat 50 μg/mL.

**Figure 5** is a graph showing the impact of several combinations of vancomycin and orlistat on the growth of *Mycobacterium tuberculosis* H37Rv. The results of Figure 5 were obtained with the BacT/Alert MP system. H37Rv 1/1: untreated and undiluted *M. tuberculosis* H37Rv strain. H37Rv 1/100: untreated and undiluted *M. tuberculosis* H37Rv strain diluted 1/100. H37Rv + vancomycin 5 μg/ml: *tuberculosis* H37Rv strain grown in presence of vancomycin 5 μg/mL. H37Rv + vancomycin 5 μg/ml + THL 5 μg/ml: *tuberculosis* H37Rv strain grown in presence of vancomycin 5 μg/mL and orlistat 5 μg/mL. H37Rv + vancomycin 5 μg/ml + THL 25 μg/ml: *tuberculosis* H37Rv strain grown in presence of vancomycin 5 μg/mL and orlistat 25 μg/mL. H37Rv + vancomycin 5 μg/ml + THL 50 μg/ml: *tuberculosis* H37Rv strain grown in presence of vancomycin 5 μg/mL and orlistat 50 μg/mL.

## EXAMPLES

The present invention and disclosure are further illustrated by the following examples. Examples concerning combinations other than vancomycin and orlistat are not according to the invention and are present for illustration purposes only.

### *Materials and Methods*

### *Bacteria and cultures*

The experiments were carried out with Wild type *M. bovis* BCG GL2 as well as *M. tuberculosis* H37Rv (wild type).
Ten clinically unrelated isolates were selected from the *M. tuberculosis* collection (n > 25000) maintained and genotyped at the Tuberculosis Center, Public Health Research Institute (PHRI), NJ. The nomenclature used for the classification of the strains was based on the *IS6110* restriction fragment length polymorphism profile and further genotyping techniques as previously described (Mathema et al., Clin. Microbiol. Rev., 2006).
Mycobacteria were grown in 7H9 medium containing 0.05% Tween 80 supplemented with 10% albumin-dextrose complex (Difco Laboratories) without agitation or on 7H11 Middlebrook agar supplemented with oleic acid-albumin-dextrose complex (Difco Laboratories).

### *Drugs*

Vancomycin, ritonavir and simvastatin were purchased from Sigma. Orlistat was purchased from Sandoz, Sigma or Cayman. Stock solutions were prepared in DNAse/RNAse free water, DMSO and DMSO, respectively for vancomycin, ritonavir and simvastatin. The stock solution of orlistat was first diluted at 5mg/mL in DMSO.

### *Drug susceptibility tests*

Drug susceptibility was investigated using three methods: the standard broth dilution method (National Committee for Clinical Laboratory Standards (NCCLS, 2003)), the standardized agar proportion method and the BacT/Alert MP method (Mycobacteria Process), according to the BioMérieux protocol (Mathys et al., Antimicrob. Agents Chemother., 2009; Werngren et al., J. Clin. Microbiol. 2006; Tortoli et al., Diagn Microbiol Infect Dis., 2000; Singh et al., J. Clin. Microbiol., 2007; and David, J. Antimicrob. Chemother. 2001).
For the standard broth (BacT/ALERT MP broth supplemented by MB/BacT reconstitution fluid) dilution susceptibility test, each "second agent" compound to be tested alone or in combination was serially diluted (0.6 ml in 0.6 ml), then tested alone or in combination with a defined amount of antibiotics selected in glycopeptides or beta-lactams (optionally further combined with a beta-lactamase inhibitor, such as clavulanate). The tubes containing the compounds alone or in combination were then inoculated with the same amount of mycobacterial strains inoculum (0.6 ml of a 1/24 diluted 1 McFarland turbidity suspension). Bacterial growth was visually inspected by turbidity. The MIC (Minimum Inhibitory Concentration) of each compound/combination was determined. MIC is defined as the lowest drug concentration that prevents visible growth of the bacterial population. A strain is considered to be resistant to a drug/combination of drugs when the growth thereof in presence of said drug/combination exceeds 1% of the growth of a non-treated inoculum of bacterial cells. The MICs of the tested drugs/combinations was determined by evaluating the bacterial turbidity in the various tubes in comparison to 1) a drug-free control tube with an undiluted identical mycobacterial inoculum and 2) a drug-free control tube with a 100-fold diluted identical bacterial inoculum, used as a 1/100 proportional growth control. Combined drugs effects were investigated using drugs at sub-MICs, according to the published Fractional Inhibitory Concentration (FIC) methodology (Odds, Antimicrobiol. Chem., 2003). Briefly, the Fractional Inhibitory Concentration of a drug A and of a drug B was evaluated as follows:

$$\text{FIC of drug A} = (\text{MIC of drug A in combination})/(\text{MIC of drug A alone});$$

$$\text{FIC of drug B} = (\text{MIC of drug B in combination})/(\text{MIC of drug B alone}).$$

For identifying any synergic activity between a drug A and a drug B, the Fractional Inhibitory Concentration Index (FICI) was evaluated as follows:

$$\text{FICI} = \text{FIC of Drug A} + \text{FIC of drug B}.$$

When FICI≤0.5, the drugs A and B are considered to be synergic. When FICI = 1, the combined effect of the drugs A and B is considered to be additive. When FICI≥4, the combined effect of the drugs A and B is considered to be antagonist. For the standard agar proportion method (NCCLS, 2003), mycobacterial strains suspensions (1 McFarland turbidity) were used to perform dilution series from $10^{-1}$ to $10^{-4}$, in the presence or absence of various concentration of orlistat and vancomycin alone or in combination. The MIC was defined as the lowest drug concentration that inhibited more than 99% of the bacterial population.

In the third method used for culturing cells, i.e. the BacT/Alert MP system, BacT/ALERT MP test bottles (11 ml) supplemented with 0.5 ml restoring fluid were inoculated with 0.1 ml water (in the drug-free control bottles) or with 0.1 ml of a drug/combination of drugs solution. Mycobacterial cultures less than 4 weeks old were used to prepare homogeneous suspension adjusted to a turbidity of a 0.5 McFarland. The same volume (0.4 ml) of this mycobacterial suspension was further inoculated into all BacT/ALERT vials. A 100-fold diluted bacterial inoculum was injected in a drug-free control vial and used as a 1/100 proportional growth control. The MIC of a drug/combination of drugs was defined as the concentration of drug/combination of drugs in a bottle flagged positive in the same time as the 1/100 control bottle. The effects of drugs tested in combination was then investigated using drugs at sub-MICs according to the previously published 'x/y' methodology (David, J. Antimicrob. Chemother., 2001). Briefly, synergy was defined as x/y < 0.5 with x being the growth index (GI) value obtained for the vial with the combination of drugs and y being the lowest GI value obtained with any of the single drug used within the combination tested. For BacT/Alert MP (Mycobacteria Process) system, test and control bottles were incubated at 37°C and the growth index (GI) values were recorded every 10 min. When the 1:100 control vial was flagged and the daily ΔGI of the 1:100 control vial reached at least 30, the GI was read for at least 1 additional day to calculate the ΔGI from the previous day. In the case of a two-drug combination, a Δx/Δy quotient of < 0.5 indicates enhanced drug action.

*Results*

Example 1: Effect of vancomycin combined to ritonavir and synergistic effect of vancomycin combined to Orlistat on *Mycobacterium bovis.*

The following example with vancomycin combined to ritonavir is not according to the invention and is present for illustration purposes only.

As exemplified in Figure 1, experiments conducted on *M. bovis* BCG according to the BacT/Alert MP system method demonstrate that the combination of vancomycin (at 5μg/mL or at 100μg/mL) with ritonavir at 100μg/mL strongly impacts the growing of the mycobacteria.

Results obtained with the combination of vancomycin at 5μg/mL with Orlistat at 50 μg/mL or at 100 μg/mL also show a strongly impact on the growing of the mycobacteria. Controls performed with vancomycin and DMSO do not appear to restrain the mycobacterial growth. Moreover, the ratio "x/y" of vancomycin 5 μg/mL combined with Orlistat 50 μg/mL is of about 0.037, thus demonstrating a highly significant synergistic effect of these two drugs.

Additional experiments shown in Figure 4 were conducted with vancomycin (at 5μg/mL) and orlistat (5μg/mL, 25μg/mL and 50μg/mL) and lead to similar results, i.e. a synergistic effect of vancomycin combined to orlistat.

In order to confirm that this growth inhibition is due to a synergistic effect, MIC and FICI were determined by the macrodilution method. As shown in Table 1, the MIC of orlistat alone is 5 μg/ml and dropped to 1.25 μg/ml when in combination with vancomycin (10 μg/ml). The FICI is thus 0.24 and confirms that vancomycin and orlistat act synergistically.

Table 1: FICI results obtain with the Checkboard method using macrodilution MIC data on *M. bovis* BCG

| Drugs | MIC | FIC | FICI |
|---|---|---|---|
| Vancomycin alone | 250 | | |
| Orlistat alone | 5 | | 0,24 |
| Orlistat with fixed 10μg/ml vancomycin | 0,6 - 1,25 | 0,184 | |
| Vancomycin with fixed 1μg/ml orlistat | 15 | 0,06 | |

Therefore, combinations of vancomycin with ritonavir or of vancomycin with orlistat are thus to be regarded as novel therapeutic compositions for treating mycobacterial infections, *a fortiori* since these drugs appear to provide a significant effect when combined at sub-MIC.

In particular, the combination of vancomycin with orlistat is to be regarded as a powerful therapeutic composition for treating mycobacterial infections since these drugs appear to provide a significant synergistic effect when combined at sub-MIC.

Example 2: Synergistic effect of vancomycin combined to orlistat on *Mycobacterium tuberculosis* H37Rv using the standard broth dilution susceptibility test.

As exemplified in Figure 3, experiments conducted on *M. tuberculosis* H37Rv according to the standard broth dilution susceptibility test method further demonstrate that the combination of vancomycin at 5μg/mL with Orlistat at 6.25, 12.5, 25, 50 or 100 μg/mL strongly impacts the growing of the mycobacteria.

Controls performed with Vancomycin and Orlistat alone demonstrate that the strong beneficial effects observed with the tested combinations of drugs are obtained for drug concentrations significantly sub-MIC. The FIC of Orlistat was evaluated to be around 0.065, the FIC of vancomycin was evaluated to be around 0.077, and as a result, the FICI of vancomycin combined with Orlistat was determined to be of 0.142 (i.e. ≤0.5). The combination of vancomycin with orlistat is thus confirmed to be significantly synergic and is thus to be regarded as novel therapeutic composition for treating mycobacterial infections, *a fortiori* since these drugs appear to provide a significant effect when combined at sub-MIC.

Example 3: Effect of vancomycin combined to ritonavir, or simvastatin and synergistic effect of vancomycin combined to orlistat on *Mycobacterium tuberculosis*.

The following examples with vancomycin combined to ritonavir or simvastatin are not according to the invention and are present for illustration purposes only.

As exemplified in Figure 2, experiments conducted on *M. tuberculosis* H37Rv according to the BacT/Alert MP system method demonstrate that the combination of vancomycin at 1 μg/mL or at 5 μg/mL with Orlistat at 50 μg/mL or at 100 μg/mL strongly impacts the growing of the mycobacteria.

Moreover, the ratio "x/y" of vancomycin 1μg/mL combined with Orlistat 50 μg/mL is of about 0.04, thus demonstrating a highly significant synergistic effect of these two drugs.

Additional experiments shown in Figure 5 were conducted with vancomycin (at 5 μg/mL) and orlistat (5 μg/mL, 25 μg/mL and 50 μg/mL) and lead to similar results, i.e. a synergistic effect of vancomycin combined to orlistat.

Similarly to the previous example, in order to confirm that this growth inhibition is due to a synergistic effect, MIC and FICI were determined by the macrodilution method. Orlistat MIC was 50 μg/ml when tested alone and fell to 1.25 μg/ml when combined with vancomycin (10 μg/ml). Based on this data, the FICI calculation showed synergism for orlistat with vancomycin (Table 2, FICI=0.236).

Table 2: FICI results obtain with the Checkboard method using macrodilution MIC data on *M. tuberculosis* H37Rv

| Drugs | MIC | FIC | FICI |
|---|---|---|---|
| Vancomycin alone | 200 - 100 | | |

(continued)

| Drugs | MIC | FIC | FICI |
|---|---|---|---|
| Orlistat alone | 50 | | 0,236 |
| Orlistat with fixed 10μg/ml vancomycin | 1,25 - 2,5 | 0,036 | |
| Vancomycin with fixed 1μg/ml orlistat | 31 | 0,2 | |

Finally, NCCLS agar proportion method was used to confirm those data. Using this method, orlistat MIC was 5 μg/ml and fell to 0.62 μg/ml with 2μg/ml vancomycin. The Checkerboard allowed us to calculate a FICI of 0.37 for orlistat with vancomycin (Table 3).

Table 3: Drug susceptibility results (MIC) obtained by the NCCLS agar proportion method and analysed by the Checkboard method for *M. tuberculosis*

| Drugs | MIC | FIC | FICI |
|---|---|---|---|
| Vancomycin alone | 40 | | |
| Orlistat alone | 5 | | |
| Orlistat with fixed 2 μg/ml vancomycin | 0,62 | 0,124 | 0,374 |
| Vancomycin with fixed 1μg/ml orlistat | 10 | 0,25 | |

This synergy clearly suggests that orlistat destabilize the outer membrane of the cell wall in order to facilitate the vancomycin action and could therefore be also active on MDR and XDR strains.

Moreover, the MIC of vancomycin alone is about 25 μg/ml (Collins and Uttley, Journal of Antimicrobial Chemotherapy. 1988, 22:857-861), which is too high to be administer to patients suffering of tuberculosis and is not indicated among potential tuberculosis treatments of the World Health Organization. However, it is shown here that vancomycin MIC fell to 2 μg/ml when combined with orlistat. At such a concentration, vancomycin may be used as an anti-tuberculosis treatment.

Further, a strong beneficial effect is also obtained with the combination of vancomycin at 5 μg/mL with simvastatin at 100 μg/mL or of vancomycin at 5 μg/mL with ritonavir at 10 μg/mL. Controls performed with vancomycin and DMSO do not appear to restrain the mycobacterial growth.

The combinations of vancomycin with ritonavir, orlistat or simvastatin are thus to be regarded as novel therapeutic compositions for treating mycobacterial infections, *a fortiori* since these drugs appear to provide a significant effect when combined at sub-MIC. The MIC of Vancomycin, Orlistat, Ritonavir and Simvastatin alone were respectively determined to be 65 μg/mL, 100μg/mL, 50-25 μg/mL and 100-50 μg/mL.

In particular, the combination of vancomycin with orlistat is to be regarded as a powerful therapeutic composition for treating mycobacterial infections since these drugs appear to provide a significant synergistic effect when combined at sub-MIC.

Similar experiments were performed on *M. tuberculosis* H37Rv with the combination of meropenem 1 μg/mL with orlistat at 50 μg/mL or at 100 μg/mL, and further optionally with amoxicillin 1.25 μg/mL and clavulanate 0.25 μg/mL.

These experiments demonstrated that a synergistic effect is obtained on *M. tuberculosis* H37Rv by the combination of meropenem 1 μg/mL with orlistat at 50 μg/mL or at 100 μg/mL, and that the said synergy is further enhanced in presence of amoxicillin 1.25 μg/mL and clavulanate 0.25 μg/mL (data not shown).

Example 4: Synergistic inhibitory effect between vancomycin and cerulenin towards MDR and XDR *M. tuberculosis* clinical isolates.

The following example with vancomycin combined to cerulenin is not according to the invention and is present for illustration purposes only.

The potential clinical use of vancomycin in combination with a cell wall-targeting drug was investigated on mycobacteria with a combination of vancomycin and cerulenin, a potent long-chain lipids synthesis inhibitor (Rastogi et al., FEMS Immunol. Med. Microbiol., 1998; Parrish et al., J. Antimicrob. Chemother., 1999). Vancomycin and cerulenin were combined at sub-MIC and tested on *M. tuberculosis* H37Rv and on MDR (multidrug resistant) and XDR (extremely drug resistant) *M. tuberculosis* clinical isolates. On *M. bovis* BCG, the combination of vancomycin (10 μg/mL) with cerulenin (0.5 μg/mL) inhibited 99% of the cell growth (data not shown). Interestingly, the combination of vancomycin (6 μg/mL) with cerulenin (1 μg/mL) was synergistically effective to inhibit the growth of 7 MDR and 3 XDR *M. tuberculosis* clinical isolates, according to the calculated x/y quotient values (see Table 4 below).

Table 4. Synergic effect between vancomycin and cerulenin towards *M. tuberculosis* multidrug-resistant clinical isolates.

| Isolates[a] | FP[b] | TN#[c] | Resistance profile[d] | | Synergic effect |
|---|---|---|---|---|---|
| | | | First line drugs | Second line drugs | |
| 1 | OO1 | 16054 | INH, RIF, EMB, STR | RMC, PAS | + (0.30) |
| 2 | BE | 17182 | INH, RIF | ETH, PAS | ++ (0.10) ($V_5C_{0.5}$) |
| 3 | W283 | 14178 | INH, RIF, EMB, PZA, STR | KAN, CAP, RFB, PAS | + (0.43) ($V_5C_{0.5}$) |
| 4 | OO1 | 18048 | INH, RIF, PZA, STR | RBT, RMC, PAS | + (0.32) |
| 5 | A7 | 6196 | INH, RIF, EMB, PZA, STR | ETH, KAN, PAS | + (0.32) |
| 6 | P | 16442 | INH, RIF, PZA, STR | RMC, PAS | + (0.20) |
| 7 | P23 | 16906 | INH, RIF, EMB, PZA, STR | ETH, RMC, PAS | + (0.25) |
| 8 | BE | 18460 | INH, RIF, EMB, STR | ETH, CYC, CIP, KAN, CAP, RFB, RMC, PAS | +++ (0.05) |
| 9 | W | 2550 | INH, RIF, EMB, PZA, STR | ETH, OFX, KAN, CYC, PAS | + (0.28) |
| 10 | HD15 | 18985 | INH, RIF, EMB, PZA, STR | CYC, CIP, OFX, KAN, AMI, CAP, RFB, RMC, PAS | + (0.13) |

[a]*Isolates 8 to 10 are XDR strains.*
[b]*FP, fingerprint name based on IS6110 typing and PHRI nomenclature.*
[c]*TN, tracking number, a PHRI unique identifier for each isolate*
[d]*Abbreviations: AMI, amikacin; CAP, capreomycin; CIP, ciprofloxacin; CYC, cycloserin; EMB, ethambutol; ETH, ethionamide; INH, isoniazid; KAN, kanamycin; OFX, ofloxacin; PAS, para-aminosalicylic acid; PZA, pyrazinamide; RBT, rifabutin; RFB, rifabutin; RIF, rifampin; RIP, rifapentine, RMC, rifamycin; STR, streptomycin.*

**BIBLIOGRAPHIE**

Baumann et al., "Cystobactamids: Myxobacterial Topoisomerase Inhibitors Exhibiting Potent Antibacterial Activity", 2014, Angew Chem Int Ed Engl., 53(52): 14605-14609;

Camacho et al., "Analysis of the phthiocerol dimycocerosate locus of Mycobacterium tuberculosis. Evidence that this lipid is involved in the cell wall permeability barrier", 2001, J Biol Chem, 276: 19845-54;

Collins and Uttley, "In-vitro activity of seventeen antimicrobial compounds against seven species of mycobacteria", 1988, Journal of Antimicrobial Chemotherapy., 22:857-861;

David, "Synergic activity of D-cycloserine and b-chloro-D-alanine against Mycobacterium tuberculosis", 2001, J. Antimicrob. Chemother., 47:203-206;

Douglas JD et al., "Analogues of thiolactomycin: potential drugs with enhanced anti-mycobacterial activity", 2002, Microbiology, 148: 3101-3109;

Jackson et al., "Inactivation of the antigen 85C gene profoundly affects the mycolate content and alters the permeability of the Mycobacterium tuberculosis cell envelope", 1999, Mol Microbiol, 31(5): 1573-1587;

Kitson et al., "Synthesis of 19-substituted geldanamycins with altered conformations and their binding to heat shock protein Hsp90", 2013, Nat. Chem., 5(4): 307-314;

Kitson et al., "Learning from nature: advances in geldanamycin- and radicicol-based inhibitors of Hsp90", 2013, J Org Chem., 78(11): 5117-41;

Marrakchi et al., "InhA, a target of the antituberculous drug isoniazid, is involved in a mycobacterial fatty acid elongation system, FAS-II", 2000, Microbiology, 146: 289-96;

Mathema et al., "Molecular epidemiology of tuberculosis: current insights", 2006, Clin. Microbiol. Rev., 19:658-685;

Mathys et al., "Molecular genetics of para-aminosalicylic acid resistance in clinical isolates and spontaneous mutants of Mycobacterium tuberculosis", 2009, Antimicrob. Agents Chemother. 53:2100-2109;

Nguyen et al., "FbpA-Dependent biosynthesis of trehalose dimycolate is required for the intrinsic multidrug resistance, cell wall structure, and colonial morphology of Mycobacterium smegmatis", 2005, J Bacteriol, 187: 6603-11;

Odds, "Synergy, antagonism, and what the chequerboard put between them", 2003 Antimicrobiol. Chem., 52 (1): 1;

Onwueme et al., "The dimycocerosate ester polyketide virulence factors of mycobacteria", 2005, Progress in Lipid Research 44: 259-302;

Parrish et al., "Antimycobacterial activity of cerulenin and its effects on lipid biosynthesis", 1999, J. Antimicrob. Chemother., 43:219-226;

Portevin et al., "A polyketide synthase catalyzes the last condensation step of mycolic acid biosynthesis in mycobacteria and related organisms", 2004, PNAS 101(1): 314-9;

Rastogi et al., "Synergistic activities of antituberculous drugs with cerulenin and trans-cinnamic acid against Mycobacterium tuberculosis", 1998, FEMS Immunol. Med. Microbiol., 21:149-157;

Singh et al., "Comparative evaluation of Löwenstein-Jensen proportion method, BacT/ALERT 3D system, and enzymatic pyrazinamidase assay for pyrazinamide susceptibility testing of Mycobacterium tuberculosis", 2007, J. Clin. Microbiol., 45: 76-80;

Takayama et al., "Pathway to synthesis and processing of mycolic acids in Mycobacterium tuberculosis", 2005, Clin. Microbiol. Reviews, 18(1):81-101;

Tortoli et al., "Comparison of Mycobacterium tuberculosis susceptibility testing performed with BACTEC 460TB (Becton Dickinson) and MB/BacT (Organon Teknika) systems", 2000, Diagn Microbiol Infect Dis., 38:83-86;

Warrier et al., "Antigen 85C inhibition restricts Mycobacterium tuberculosis growth through disruption of cord factor biosynthesis", 2012, Antimicrob Agents Chemother., 56(4): 1735-43;

Werngren et al., "Evaluation of a novel kit for use with the BacT/ALERT 3D system for drug susceptibility testing of Mycobacterium tuberculosis", 2006, J. Clin. Microbiol. 44: 2130-2132.

**Claims**

1. A composition comprising vancomycin and orlistat.

2. The composition according to claim 1, further comprising at least one additional therapeutic agent.

3. The composition according to claim 2, wherein said at least one additional therapeutic agent is selected in the group consisting of at least one of a glycopeptide antibiotic, a beta-lactam, cycloserine, a RNA synthesis inhibitor, a DNA replication inhibitor, a nucleoside antibiotic, an inhibitor of protein synthesis, an inhibitor of ATP synthesis, an inhibitor of dihydrofolate reductase, a lipase inhibitor, a statin, a protease inhibitor, a heat shock protein inhibitor, a mycolic acid or a fatty acid synthesis inhibitor, an inhibitor of ACP-domain containing proteins, an Ag85C inhibitor, a disulphide bridge inhibitor and a trisubstituted imidazole.

4.  The composition according to any one of claims 1 to 3, further comprising at least one additional potentiating agent, preferably selected from beta-lactamase inhibitors, wherein said potentiating agent increases the mycobacteriostatic and/or mycobactericidal properties of said composition.

5.  The composition according to claim 4, comprising clavulanate and/or ampicillin as additional potentiating agent.

6.  A pharmaceutical composition comprising the composition as defined in any one of claims 1 to 5 and one or more pharmaceutically acceptable excipients.

7.  A kit of parts comprising vancomycin and orlistat as described in any one of claims **1** to **3**, and further optionally comprising the at least one additional potentiating agent as described in any one of claims 4 and 5 in a single or in separate vials, together with suitable pharmaceutical excipients, and wherein vancomycin and orlistat, and the optionally at least one additional potentiating agent, are either dissolved or suspended in an acceptable carrier, preferably an aqueous carrier.

8.  The composition according to any one of claims 1 to 5, the pharmaceutical composition according to claim 6 or the kit of parts according to claim 7 for use as a medicament.

9.  The composition according to any one of claims 1 to 5, the pharmaceutical composition according to claim 6 or the kit of parts according to claim 7 for use in the treatment of a mycobacterial infection, preferably tuberculosis.

**Patentansprüche**

1.  Zusammensetzung, die Vancomycin und Orlistat umfasst.

2.  Zusammensetzung nach Anspruch 1, die weiter mindestens ein zusätzliches therapeutisches Mittel umfasst.

3.  Zusammensetzung nach Anspruch 2, wobei das mindestens eine zusätzliche therapeutische Mittel ausgewählt ist aus der Gruppe bestehend aus mindestens einem aus einem Glykopeptid-Antibiotikum, einem Beta-Lactam, Cycloserin, einem RNA-Synthesehemmer, einem DNA-Replikationshemmer, einem Nucleosid-Antibiotikum, einem Proteinsynthesehemmer, einem ATP-Synthesehemmer, einem Dihydrofolatreduktasehemmer, einem Lipasehemmer, einem Statin, einem Proteasehemmer, einem Hitzeschockproteinhemmer, einem Mycolsäure- oder einem Fettsäure-Synthesehemmer, einem Hemmer von ACP-Domänen-haltigen Proteinen, einem Ag85C-Hemmer, einem Disulfidbrückenhemmer und einem trisubstituierten Imidazol.

4.  Zusammensetzung nach einem der Ansprüche 1 bis 3, die weiter mindestens ein zusätzliches potenzierendes Mittel umfasst, vorzugsweise ausgewählt aus Beta-Lactamasehemmern, wobei das potenzierende Mittel die mykobakteriostatischen und/oder mykobakteriziden Eigenschaften der Zusammensetzung erhöht.

5.  Zusammensetzung nach Anspruch 4, die Clavulanat und/oder Ampicillin als zusätzliches potenzierendes Mittel umfasst.

6.  Pharmazeutische Zusammensetzung, die die Zusammensetzung wie in einem der Ansprüche 1 bis 5 definiert und einen oder mehrere pharmazeutisch verträgliche Hilfsstoffe umfasst.

7.  Teilesatz, der Vancomycin und Orlistat umfasst, wie in einem der Ansprüche **1** bis **3** beschrieben, und weiter wahlweise das mindestens eine zusätzliche potenzierende Mittel, wie in einem der Ansprüche **4** und **5** beschrieben, in einem einzigen oder in getrennten Fläschchen, zusammen mit geeigneten pharmazeutischen Hilfsstoffen umfasst, und wobei Vancomycin und Orlistat, und das wahlweise mindestens eine zusätzliche potenzierende Mittel in einem verträglichen Träger, vorzugsweise einem wässrigen Träger, entweder gelöst oder suspendiert sind.

**8.** Zusammensetzung nach einem der Ansprüche **1** bis **5,** pharmazeutische Zusammensetzung nach Anspruch **6,** oder Teilesatz nach Anspruch 7 zur Verwendung als Arzneimittel.

**9.** Zusammensetzung nach einem der Ansprüche **1** bis **5,** pharmazeutische Zusammensetzung nach Anspruch **6,** oder Teilesatz nach Anspruch 7 zur Verwendung in der Behandlung einer mykobakteriellen Infektion, vorzugsweise Tuberkulose.

**Revendications**

**1.** Une composition comprenant de la vancomycine et de l'orlistat.

**2.** La composition selon la revendication **1,** comprenant en outre au moins un agent thérapeutique additionnel.

**3.** La composition selon la revendication **2**, dans laquelle ledit au moins un agent thérapeutique additionnel est sélectionné parmi le groupe consistant en au moins un antibiotique glycopeptide, un bêta-lactamine, la cyclosérine, un inhibiteur de la synthèse de l'ARN, un inhibiteur de la réplication de l'ADN, un antibiotique nucléosidique, un inhibiteur de la synthèse protéique, un inhibiteur de la synthèse de l'ATP, un inhibiteur de la dihydrofolate réductase, un inhibiteur de lipase, une statine, un inhibiteur de protéase, un inhibiteur de protéine de choc thermique, un inhibiteur de la synthèse d'acide mycolique ou d'acide gras, un inhibiteur de protéines contenant un domaine ACP, un inhibiteur d'Ag85c, un inhibiteur de pont disulfure, et un imidazole trisubstitué.

**4.** La composition selon l'une quelconque des revendications **1** à **3,** comprenant en outre au moins un agent potentialisateur additionnel, de préférence sélectionné parmi les inhibiteurs de bêta-lactamase, dans laquelle ledit agent potentialisateur additionnel augmente les propriétés mycobactériostatiques et/ou mycobactéricides de ladite composition.

**5.** La composition selon la revendication **4,** comprenant du clavulanate et/ou de l'ampicilline comme agent potentialisateur additionnel.

**6.** Une composition pharmaceutique comprenant la composition telle que définie selon l'une quelconque des revendications **1** à **5** et un ou plusieurs excipient(s) pharmacologiquement acceptable(s).

**7.** Un kit d'éléments comprenant de la vancomycine et de l'orlisat comme décrit dans selon l'une quelconque des revendications **1** à **3**, et comprenant en outre optionnellement ledit au moins un agent potentialisateur additionnel comme décrit dans l'une quelconque des revendications **4** et **5** dans un flacon unique ou dans des flacons séparés, conjointement avec des excipients pharmacologiques acceptables, et dans lequel la vancomycine et l'orlistat, et l'au moins un agent potentialisateur additionnel optionnel, sont soit dissouts soit en suspension dans un support acceptable, de préférence un support aqueux.

**8.** La composition selon l'une quelconque des revendications **1** à **5,** la composition pharmaceutique selon la revendication **6** ou le kit d'éléments selon la revendication **7** pour une utilisation comme médicament.

**9.** La composition selon l'une quelconque des revendications **1** à **5,** la composition pharmaceutique selon la revendication **6** ou le kit d'éléments selon la revendication **7** pour une utilisation dans le traitement d'une infection mycobactérienne, de préférence la tuberculose.

**FIG. 1**

**FIG. 2**

**FIG. 3**

EP 3 237 011 B1

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03082911 A
- WO 2008129146 A
- WO 2007069089 A

### Non-patent literature cited in the description

- **PORTEVIN et al.** *PNAS,* 2004, vol. 101 (1), 314-9
- **NGUYEN et al.** *J Bacteriol,* 2005, vol. 187, 6603-11
- **CAMACHO et al.** *J Biol Chem,* 2001, vol. 276, 19845-54
- **JACKSON et al.** *Mol Microbiol,* 1999, vol. 31 (5), 1573-1587
- **MARRAKCHI et al.** *Microbiology,* 2000, vol. 146, 289-96
- *Journal of Antimicrobial Chemotherapy,* 1988, vol. 22, 857-861
- **MAJUMDAR et al.** *Indian J Tuberc.,* January 2011, vol. 58 (1), 4-10
- **WANKHADE et al.** *Indian J Tuberc.,* July 2012, vol. 59 (3), 156-61
- **ODDS.** *Antimicrobiol. Chem.,* 2003
- **DAVID.** *J. Antimicrob. Chemother.,* 2001
- **DOUGLAS JD et al.** *Microbiology,* 2002, vol. 148, 3101-3109
- **TAKAYAMA et al.** *Clin. Microbiol. Reviews,* 2005, 81-101
- **ONWUEME et al.** *Progress in Lipid Research,* 2005, vol. 44, 259-302
- Harrison's Principles of Internal Medicine. 2005, 953-966
- **MATHEMA et al.** *Clin. Microbiol. Rev.,* 2006
- **MATHYS et al.** *Antimicrob. Agents Chemother.,* 2009
- **WERNGREN et al.** *J. Clin. Microbiol.,* 2006
- **TORTOLI et al.** *Diagn Microbiol Infect Dis.,* 2000
- **SINGH et al.** *J. Clin. Microbiol.,* 2007
- **COLLINS ; UTTLEY.** *Journal of Antimicrobial Chemotherapy,* 1988, vol. 22, 857-861
- **RASTOGI et al.** *FEMS Immunol. Med. Microbiol.,* 1998
- **PARRISH et al.** *J. Antimicrob. Chemother.,* 1999
- **BAUMANN et al.** Cystobactamids: Myxobacterial Topoisomerase Inhibitors Exhibiting Potent Antibacterial Activity. *Angew Chem Int Ed Engl.,* 2014, vol. 53 (52), 14605-14609
- **CAMACHO et al.** Analysis of the phthiocerol dimycocerosate locus of Mycobacterium tuberculosis. Evidence that this lipid is involved in the cell wall permeability barrier. *J Biol Chem,* 2001, vol. 276, 19845-54
- **COLLINS AND UTTLEY.** In-vitro activity of seventeen antimicrobial compounds against seven species of mycobacteria. *Journal of Antimicrobial Chemotherapy.,* 1988, vol. 22, 857-861
- **DAVID.** Synergic activity of D-cycloserine and b-chloro-D-alanine against Mycobacterium tuberculosis. *J. Antimicrob. Chemother.,* 2001, vol. 47, 203-206
- **DOUGLAS JD et al.** Analogues of thiolactomycin: potential drugs with enhanced anti-mycobacterial activity. *Microbiology,* 2002, vol. 148, 3101-3109
- **JACKSON et al.** Inactivation of the antigen 85C gene profoundly affects the mycolate content and alters the permeability of the Mycobacterium tuberculosis cell envelope. *Mol Microbiol,* 1999, vol. 31 (5), 1573-1587
- **KITSON et al.** Synthesis of 19-substituted geldanamycins with altered conformations and their binding to heat shock protein Hsp90. *Nat. Chem.,* 2013, vol. 5 (4), 307-314
- **KITSON et al.** Learning from nature: advances in geldanamycin- and radicicol-based inhibitors of Hsp90. *J Org Chem.,* 2013, vol. 78 (11), 5117-41
- **MARRAKCHI et al.** InhA, a target of the antituberculous drug isoniazid, is involved in a mycobacterial fatty acid elongation system, FAS-II. *Microbiology,* 2000, vol. 146, 289-96
- **MATHEMA et al.** Molecular epidemiology of tuberculosis: current insights. *Clin. Microbiol. Rev.,* 2006, vol. 19, 658-685

- **MATHYS et al.** Molecular genetics of para-aminosalicylic acid resistance in clinical isolates and spontaneous mutants of Mycobacterium tuberculosis. *Antimicrob. Agents Chemother.,* 2009, vol. 53, 2100-2109
- **NGUYEN et al.** FbpA-Dependent biosynthesis of trehalose dimycolate is required for the intrinsic multidrug resistance, cell wall structure, and colonial morphology of Mycobacterium smegmatis. *J Bacteriol,* 2005, vol. 187, 6603-11
- **ODDS.** Synergy, antagonism, and what the chequerboard put between them. *Antimicrobiol. Chem.,* 2003, vol. 52 (1), 1
- **ONWUEME et al.** The dimycocerosate ester polyketide virulence factors of mycobacteria. *Progress in Lipid Research,* 2005, vol. 44, 259-302
- **PARRISH et al.** Antimycobacterial activity of cerulenin and its effects on lipid biosynthesis. *J. Antimicrob. Chemother,* 1999, vol. 43, 219-226
- **PORTEVIN et al.** A polyketide synthase catalyzes the last condensation step of mycolic acid biosynthesis in mycobacteria and related organisms. *PNAS,* 2004, vol. 101 (1), 314-9
- **RASTOGI et al.** Synergistic activities of antituberculous drugs with cerulenin and trans-cinnamic acid against Mycobacterium tuberculosis. *FEMS Immunol. Med. Microbiol.,* 1998, vol. 21, 149-157
- **SINGH et al.** Comparative evaluation of Löwenstein-Jensen proportion method, BacT/ALERT 3D system, and enzymatic pyrazinamidase assay for pyrazinamide susceptibility testing of Mycobacterium tuberculosis. *J. Clin. Microbiol.,* 2007, vol. 45, 76-80
- **TAKAYAMA et al.** Pathway to synthesis and processing of mycolic acids in Mycobacterium tuberculosis. *Clin. Microbiol. Reviews,* 2005, vol. 18 (1), 81-101
- **TORTOLI et al.** Comparison of Mycobacterium tuberculosis susceptibility testing performed with BACTEC 460TB (Becton Dickinson) and MB/BacT (Organon Teknika) systems. *Diagn Microbiol Infect Dis.,* 2000, vol. 38, 83-86
- **WARRIER et al.** Antigen 85C inhibition restricts Mycobacterium tuberculosis growth through disruption of cord factor biosynthesis. *Antimicrob Agents Chemother.,* 2012, vol. 56 (4), 1735-43
- **WERNGREN et al.** Evaluation of a novel kit for use with the BacT/ALERT 3D system for drug susceptibility testing of Mycobacterium tuberculosis. *J. Clin. Microbiol.,* 2006, vol. 44, 2130-2132